# EUROPEAN PATENT SPECIFICATION

(11) **EP 2 536 434 B1**
(45) Date of publication and mention of the grant of the patent: **20.04.2016**
(21) Application number: 11703642.6
(22) Date of filing: 03.02.2011
(51) Int. Cl.: A61K 47/48, C07K 1/22

(54) **PURIFICATION METHOD**
REINIGUNGSVERFAHREN
PROCÉDÉ DE PURIFICATION

(30) Priority: 19.02.2010 US 306101 P; 16.02.2010 EP 10153713
(43) Date of publication of application: 26.12.2012
(73) Proprietor: Novo Nordisk A/S, 2880 Bagsværd (DK)
(72) Inventor: BUCHARDT, Jens, 2820 Gentofte (DK)
(86) International application number: PCT/EP2011/051570
(87) International publication number: WO 2011/101261

(56) References cited:
- WO-A1-2010/015668
- WO-A2-2005/012347
- WO-A2-2005/027978
- WO-A2-2005/028516
- US-A- 4 411 832
- US-A- 5 576 175
- US-A1- 2003 069 395
- US-A1- 2007 203 058
- JUNG-LAW LIN, YIH-SHOU HSIEH, SHU-CHEN CHU: "The Isolation and Purification of Pre-S2 Containing Hepatitis B Virus Surface Antigen by Chemical Affinity Chromatography", APPLIED BIOCHEMISTRY AND BIOTECHNOLOGY, vol. 15, no. 3, 1987, pages 255-263, XP002663228,
- SASSON K ET AL: "Engineering prolonged-acting prodrugs employing an albumin-binding probe that undergoes slow hydrolysis at physiological conditions", JOURNAL OF CONTROLLED RELEASE, ELSEVIER, AMSTERDAM, NL LNKD- DOI:10.1016/J.JCONREL.2009.10.028, vol. 142, no. 2, 30 October 2009 (2009-10-30), pages 214-220, XP026905283, ISSN: 0168-3659 [retrieved on 2009-10-30]
- VRETBLAD ET AL: "Immobilization of ligands for biospecific affinity chromatography VIA their hydroxyl groups. the cyclohexaamylose-beta-amylase system", FEBS LETTERS, ELSEVIER, AMSTERDAM, NL LNKD- DOI:10.1016/0014-5793(74)80431-X, vol. 47, no. 1, 1 October 1974 (1974-10-01), pages 86-89, XP025601449, ISSN: 0014-5793 [retrieved on 1974-10-01]

## Description

### Field of the Invention

The present application relates to protein purification. In particular, the invention relates to the purification of proteins that are conjugates to albumin binder moieties. This has particular utility during the preparation of such conjugates where it is desired to isolate the conjugates from any parent protein that is not conjugated to an albumin binder.

### Background to the Invention

It is known that the covalent attachment of albumin binder moieties to proteins can give rise to increased *in vivo* half-life for the proteins. Such protein-albumin binder conjugates therefore have utility in the *in vivo* administration of therapeutic, prophylactic or diagnostic proteins and the presence of the albumin binder means that the *in vivo* effectiveness of the protein can be increased.

When protein-albumin binder conjugates are produced, it is desirable to have an efficient method for separating the conjugates from any parent protein that is not conjugated to an albumin binder. The ability to achieve such efficient purification can be problematic in practice, particularly with larger proteins.

Since the albumin binders commonly contain a fatty acid component, separation is often approached on the basis of hydrophobicity (e.g. HPLC). However, this is not practical in the case of larger proteins such as human growth hormone (hGH) and Factor VIII (FVIII) due to the instability of such proteins under these conditions.

The use of ion exchange chromatography is also difficult, since the conjugates contain highly hydrophobic groups which lead to a tendency of the conjugates to irreversibly bind to the ion exchange column, or to precipitate, especially in the presence of a high salt concentration, e.g. during the elution step.

There therefore remains a need for an efficient method for purifying protein-albumin binder conjugates, particularly where the protein of interest is a larger protein such as hGH or FVIII.

### Summary of the Invention

The present invention provides methods that allow for the purification of protein-albumin binder conjugates. In particular, the methods of the invention allow for the purification of such protein-albumin binder conjugates from a mixture that also comprises the protein in unbound form, i.e. the same protein that is present in the conjugates, but in a form where the protein is not conjugated to the albumin binder.

Accordingly, the present invention provides a method for purification of a protein that is conjugated to an albumin binding moiety from a mixture comprising (i) said protein in said conjugated form and (ii) said protein in a form that is not conjugated to said albumin-binding moiety, the method comprising:
(a) providing a solid support comprising a substance capable of specifically binding to the albumin binding moiety, wherein said substance is selected from the group consisting of albumin and cyclodextrin;
(b) contacting said solid support of (a) with said mixture comprising protein and conjugated protein under suitable conditions for binding of the albumin binding moiety to the substance as defined in (a);
(c) eluting components bound to the solid support.

The elution of step (c) comprises contacting the solid support with a substance capable of binding to the albumin binding moiety, such as a substance is capable of competitively binding to the albumin binding moiety. A suitable substance may be, for example, cyclodextrin. Said substance may be applied to said solid support in a gradient of increasing concentration in step (c).

The substance capable of specifically binding to the albumin binding moiety in step (a) is selected from the group consisting of albumin or cyclodextrin.

The protein may be selected from Factor VIII, growth hormone, Factor VII, FIX, GLP-1, insulin, or a variant form of any thereof.

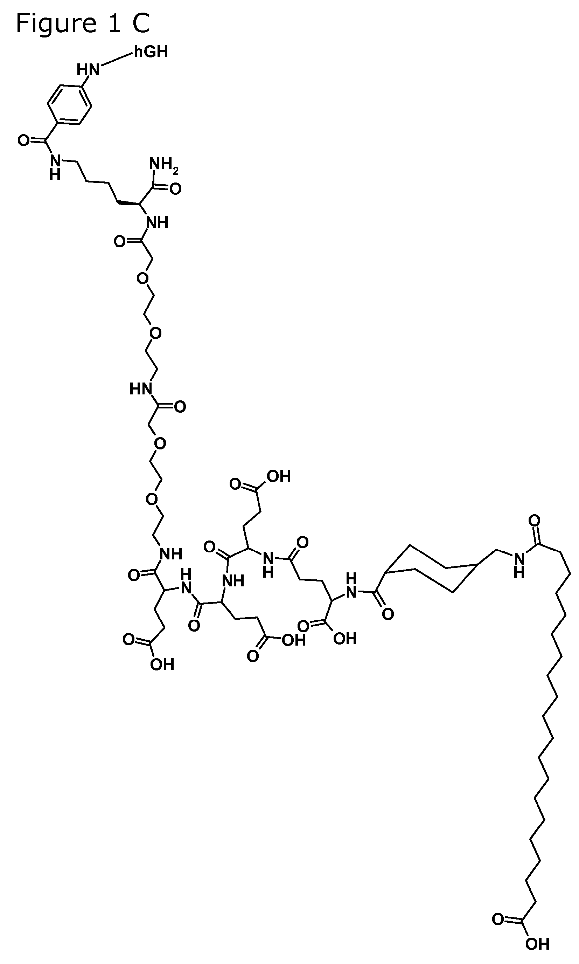

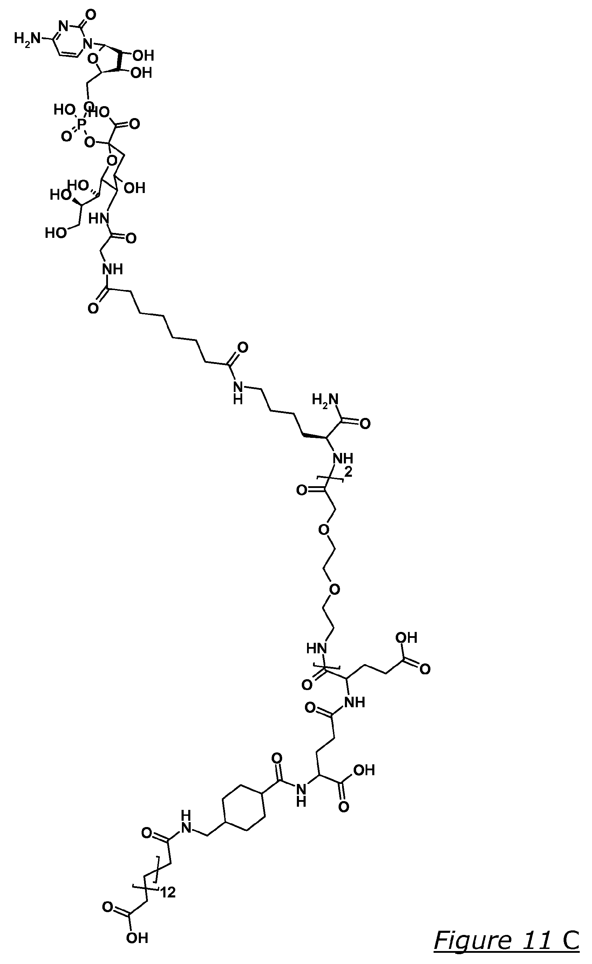

The albumin binding moiety may extend the half life of the protein *in vivo.* The albumin binding moiety may comprise a fatty acid or fatty acid derivative. The albumin binding moiety may be as defined in Figure 1C or Figure 11C.

### Brief Description of the Figures

Figure 1 shows the results of the chromatography protocol in Example 4. Figure 1A shows the elution gradient protocol that was used and Figure 1B shows the resultant chromatogram.
Figure 2 shows the results of TOF-ESI MS of fraction 1 (Figure 2A) and fraction 7 (Figure 2B) from Figure 1B. Fraction 1 was identified as unmodified hGH and fraction 7 was identified as albumin binder-hGH conjugate.
Figure 3 shows the chromatograms results from Example 5. Figure 3A = hGH sample, Figure 3B = sample: mixture of hGH and hGH-albumin binder conjugate (005). No separation between unmodified hGH and hGH albumin binder conjugates was observed.

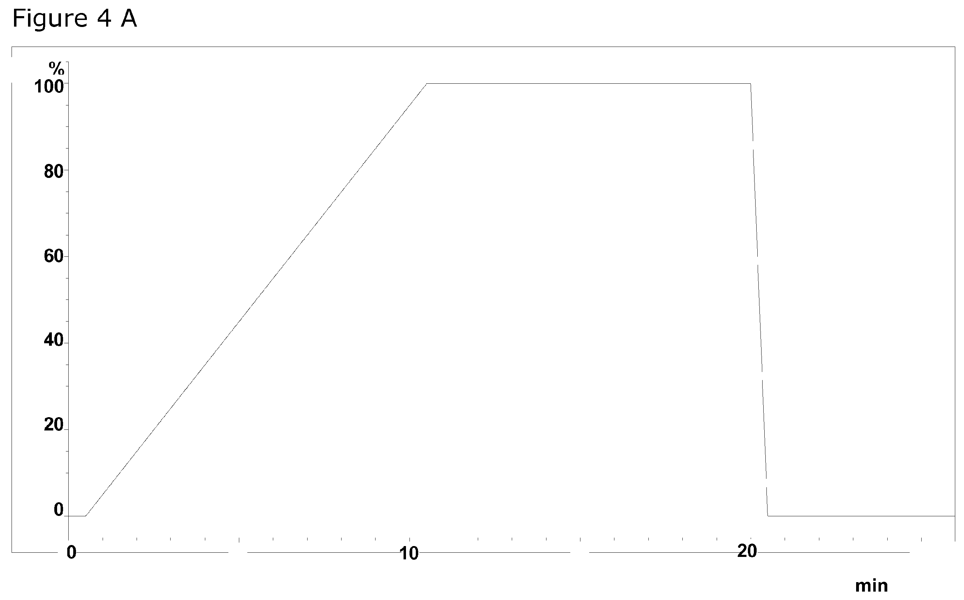

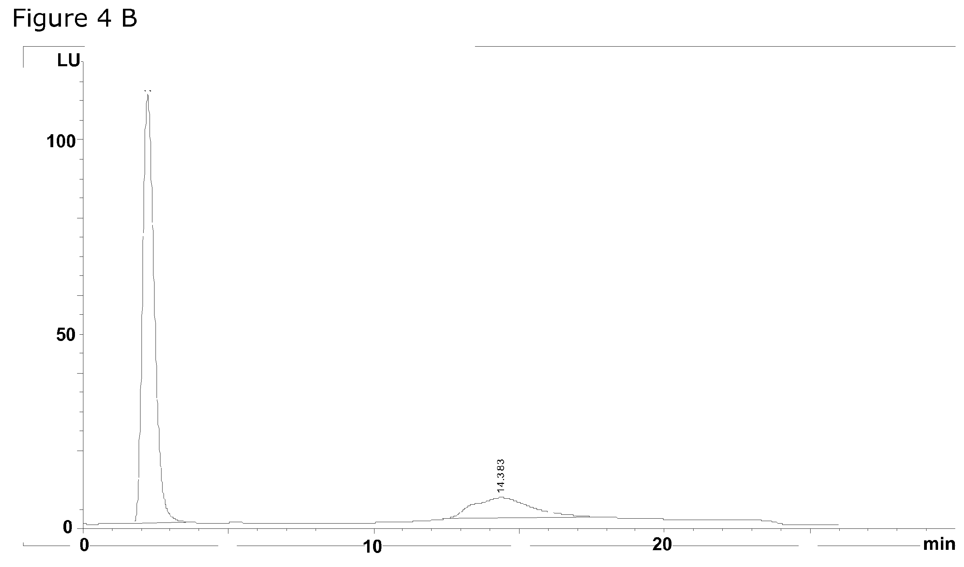

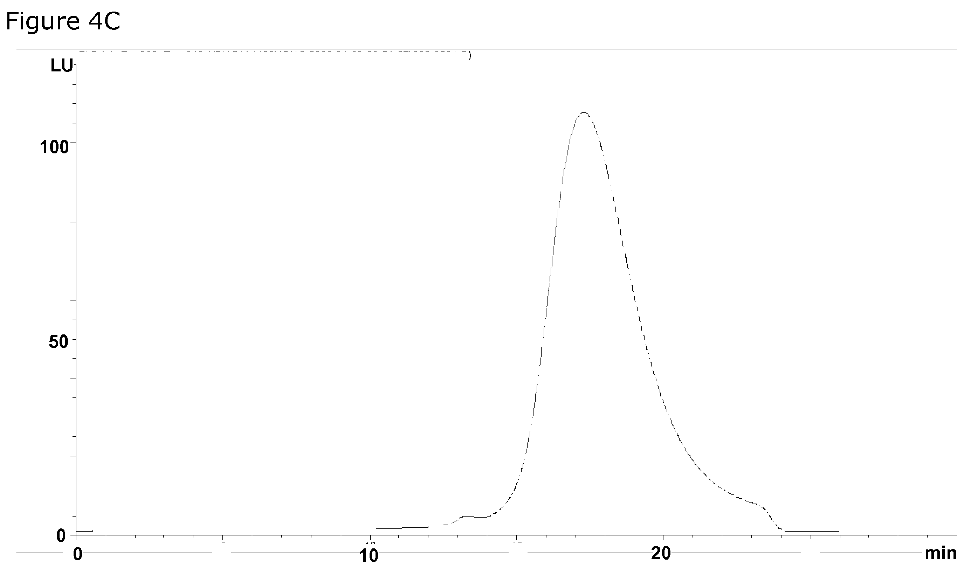

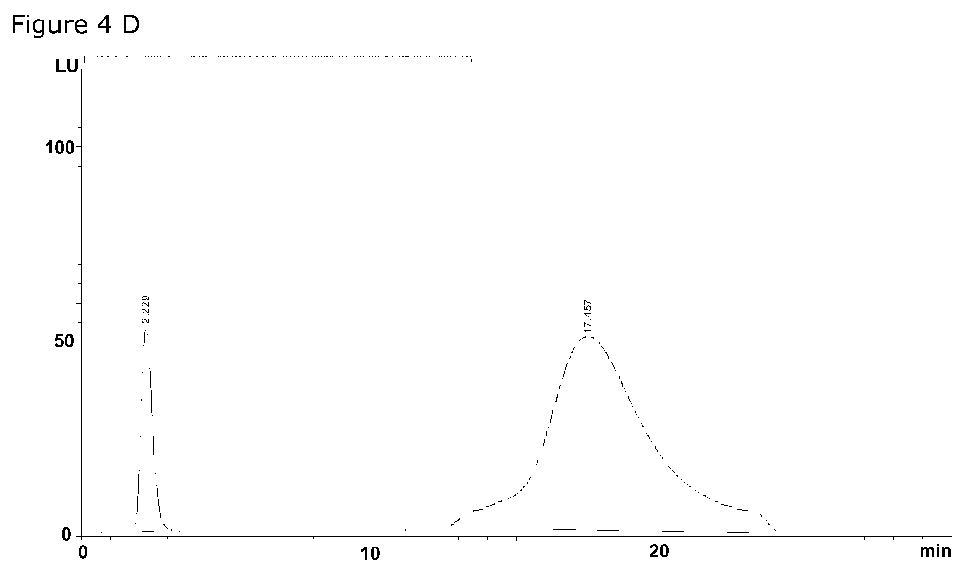

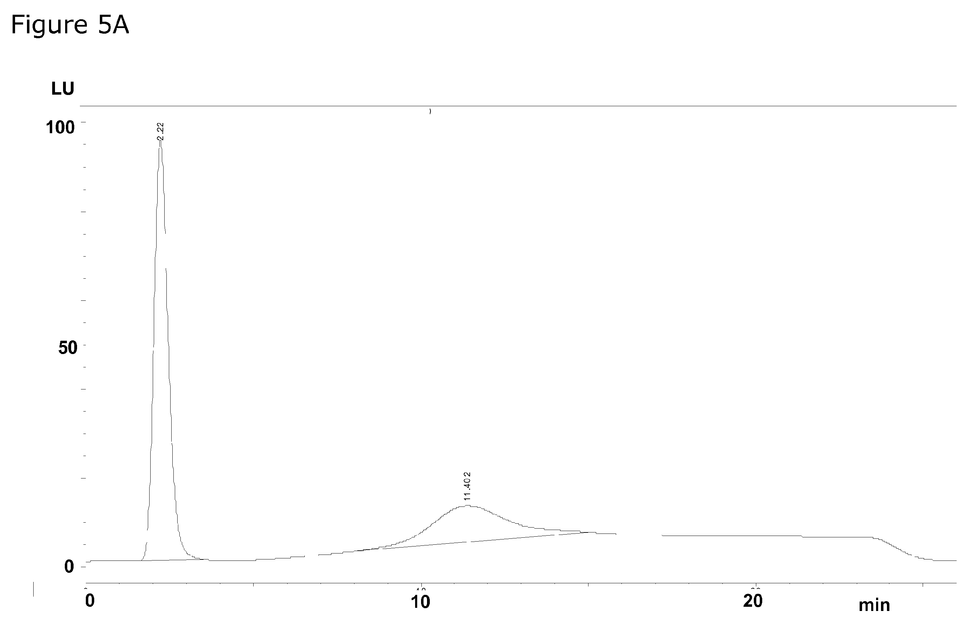

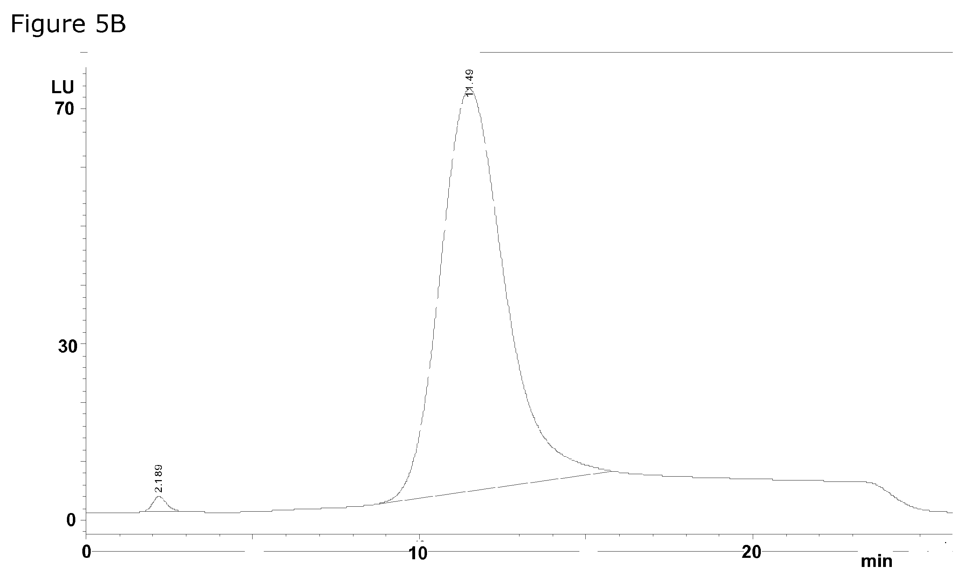

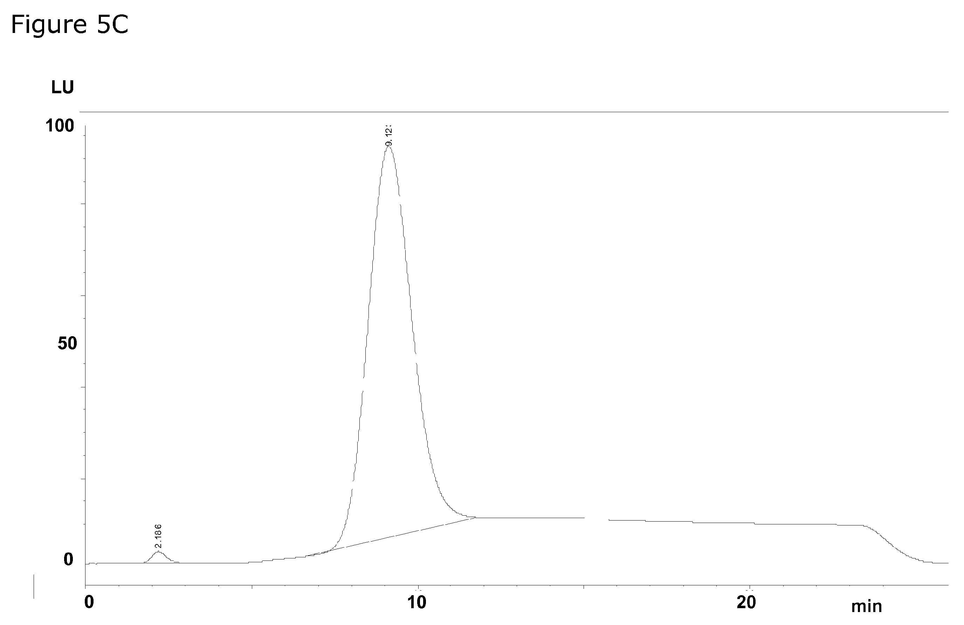

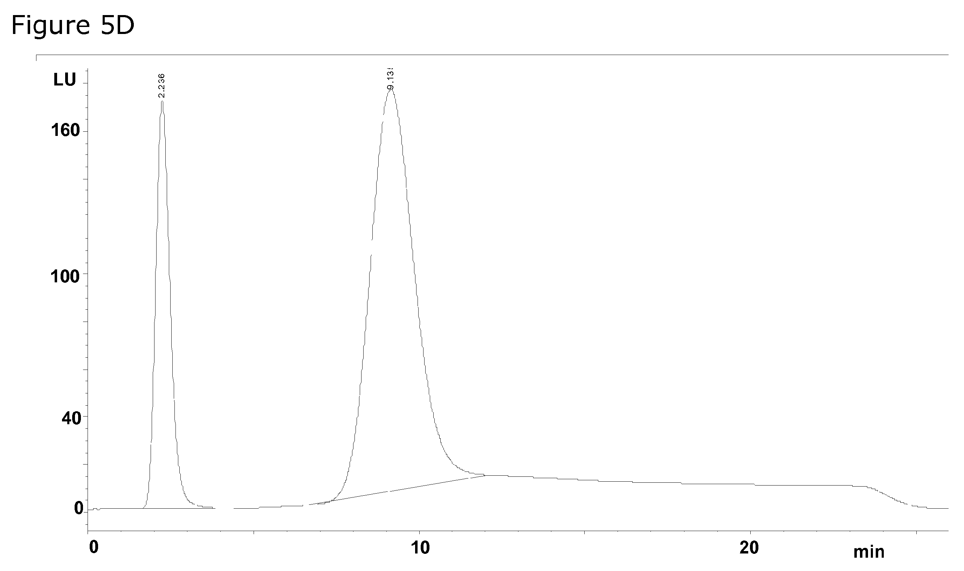

Figures 4 and 5 show the chromatograms obtained in Example 6. Figure 4A shows the elution gradient profile used in the first part of the experiment. Figure 4B = hGH (10µl of 2 mg/ml). Figure 4C = hGH-albumin binder conjugate (10µl of 2 mg/ml). Figure 4D = co-injection. Figure 5 shows the results from experiments in which cyclodextrin was used in the B buffer. Figure 5A = hGH (5µl of 2 mg/ml), 10 mM HPCD in B-buffer. A little carry over of hGH-albumin binder was seen. Figure 5B = hGH-albumin binder conjugate (5µl of 2 mg/ml), 10mM HPCD in B buffer. Figure 5C = hGH-albumin binder conjugate (5µl of 2 mg/ml), 20mM HPCD in B buffer. Figure 5D = co-injection: 20µl of 1 mg/ml of each of hGH and hGH-albumin binder conjugate, 20mM HPCD in B buffer.
Figure 6 shows the chromatograms resulting from Example 7. Figure 6A: the lowest trace (blue) is a blank. The next highest trace (red) is the Factor VIII molecule used. The top trace (green) is the albumin binder conjugate, crude reaction mixture after coupling an albumin binder to the N-glycans of the Factor VIII molecule using a sialyltransferase (see Example 12). Figure 6B (top) = chromatogram, (bottom) = elution gradient profile
Figure 7 shows the SDS-PAGE of the fractions identified in Figure 6B. Lanes 1-7 = fractions 1, 2, 4, 6, 7, 8, 9 respectively. Lane 8 = N8. Lane 9 = FVIII -albumin binder conjugate, Crude reaction mixture (17754-152-1). Lanes 10-15 = fractions 10-15 respectively
Figure 8 shows the chromatograms obtained using preparations 158-1 and 158-II. Top: N8; 2^{nd} row: 158-1; 3^{rd} row: 158-II; 4^{th} row: elution gradient profile.
Figures 9 and 10 show the results of Example 9. Figure 9A = analytical run. Top = chromatogram, bottom = gradient elution profile. Figure 9B shows the preparative run. Top = chromatogram, bottom = gradient elution profile. Figure 10A = protein content in each fraction from Figure 9B as measured by Nanodrop (A280, E 1% 14.6). Figure 10B = SDS-PAGE of fractions from 10A. Lane 1 = N8; lane 2 = reactionmix. 3 hours; Lanes 3-9 = fractions 3, 4, 5, 9, 10, 11 and 12 respectively; lane 10 = mix fraction 4 + fraction 10.
Figure 11A shows CD column chromatography of wt B-domain deleted FVIII modified with substrate 1 and ST3Gal-I selective for the O-glycan. Trace marked 1: UV 280 nm, trace marked 2: step gradient. Figure 11B shows traces for related experiments. N8 = wt B-domain deleted FVIII, 17754-235-III = isolated product modified with substrate 1 on the O-glycan. Figure 11C gives the structure of Substrate 1 as used in the Examples.
Figure 12 shows the results from Example 12. A = results using buffer system 1. B = results using buffer system 2. C = results using buffer system 3.
Figure 13 shows the results from Example 13.
Figure 14 shows the results from example 14
Figure 15 shows the results from example 15

### Detailed Description of the Invention

The present invention is based on the development of methods for purification of protein-albumin binder conjugates. The methods of the invention make use of an affinity chromatography approach. However, the present methods overcome a number of disadvantages of existing affinity chromatography methods which would make them unsuitable for use in purifying protein-albumin binder conjugates where the protein is a larger molecule such as hGH or FVIII.

Affinity exchange columns are available that employ immobilised albumin. However, these columns are based on silica and require high (e.g. 35-40%) concentrations of isopropanol in the eluent. Such high levels of isopropanol can be detrimental to many therapeutic proteins such as FVIII, meaning that this approach is not suitable in the present case. An alternative approach would be to include a low concentration of octanoic acid in the eluent. However, this approach is also considered to be unsuitable since it gives rise to a gradual loss of column efficiency.

The present invention instead utilises a mild and efficient affinity chromatography approach. The methods described herein allow for the efficient and specific purification of protein-albumin binder conjugates but avoid the deleterious effects of other approaches by allowing the use of only mild buffer systems which are compatible with sensitive proteins such as hGH and FVIII.

### Proteins

The present invention relates generally to proteins that are bound to albumin binders. Any protein may be used in such a method.

The methods described herein may have particular utility in relation to proteins that are intended for *in vivo* administration. For example, the methods may have utility in relation to proteins that have therapeutic, prophylactic or diagnostic utility. A suitable protein may therefore be a protein that it is desired to administer to an animal such as a human. The protein may be administered for any reason. Particularly relevant are proteins where there is a desire to maintain or improve the stability or half-life of the protein *in vivo* after it has been administered.

The present methods also address particular problems with other purification methods when larger proteins are used. By a "larger protein" is meant, for example, a protein of greater than 30 amino acids, greater than 50 amino acids or greater than 100 amino acids. Examples of such proteins include hormones such as growth hormone, insulin or GLP-1 and blood coagulation factors such as Factor VII or Factor VIII. The protein may be a human protein. For example, the protein may be human growth hormone, human insulin or human FVIII. The protein may be a recombinantly produced protein. The protein may be a naturally occurring form of a protein or a modified form of a protein.

For example, Factor VIII (FVIII) is a large, complex glycoprotein that primarily is produced by hepatocytes. FVIII consists of 2351 amino acids, including signal peptide, and contains several distinct domains, as defined by homology (see SEQ ID NO: 1). There are three A-domains, a unique B-domain, and two C-domains. The domain order can be listed as NH2-A1-A2-B-A3-C1-C2-COOH. FVIII circulates in plasma as two chains, separated at the B-A3 border. The chains are connected by bivalent metal ion-bindings. The A1-A2-B chain is termed the heavy chain (HC) while the A3-C1-C2 is termed the light chain (LC).

Endogenous Factor VIII molecules circulate *in vivo* as a pool of molecules with B domains of various sizes. It is believed that *in vivo* a gradual enzymatic removal of the B domain results in a pool of molecules with B-domains of various sizes. It is generally believed that cleavage at position 740, by which the last part of the B-domain is removed, occurs in connection with thrombin activation. However, it cannot be ruled out that a Factor VIII variant in which e.g. the cleavage site at position 740 has been impaired may be active.

"Factor VIII" or "FVIII" as used herein thus refers to a human plasma glycoprotein that is a member of the intrinsic coagulation pathway and is essential to blood coagulation. "Native FVIII" refers to the full length human FVIII molecule as shown in SEQ ID NO: 1 (amino acid 1-2332). The B-domain spans amino acids 741-1648 in this sequence

The Factor VIII molecule for use according to the present invention may be a native form of FVIII or a variant form of such a native FVIII molecule. The Factor VIII molecule may be a naturally occurring variant form of such a molecule, such as a form in which the B domain has been altered or truncated. The Factor VIII molecule may be an artificially generated FVIII molecule based on the native FVIII sequence.

A FVIII molecule for use in accordance with the present invention may be a B domain truncated Factor VIII molecule wherein the remaining domains correspond closely to the sequence as set forth in amino acid nos 1-740 and 1649-2332 in the native FVIII sequence of SEQ ID NO: 1. A FVIII molecule for use in accordance with the present invention may alternatively or additionally comprise one or more alterations within the vWF binding region between residues 1670-1684. Such a molecule may, for example, comprise amino acids 1-740 and 1649-2332 of the native sequence or may comprise 1-740, 1649-1670 and 1684-2332 of the native sequence.

FVIII molecules or B domain truncated FVIII molecules according to the invention may differ slight from the sequence set forth in SEQ ID NO 1, meaning that the sequence or the sequence of the remaining domains (i.e. the three A-domains and the two C-domains) may differ slightly e.g. about 1%, 2%, 3%, 4% or 5% from the native amino acid sequence (e.g. as set forth in SEQ ID NO 1) due to the fact that mutations can be introduced in order to e.g.reduce vWF binding capacity. Furthermore, it is plausible that amino acid modifications (substitutions, deletions, etc.) are introduced other places in the molecule in order to modify the binding capacity of Factor VIII with various other components such as e.g. LRP, various receptors, other coagulation factors, cell surfaces, introduction and/or abolishment of glycosylation sites, etc.

Factor VIII molecules according to the present invention have Factor VIII activity, meaning the ability to function in the coagulation cascade in a manner functionally similar or equivalent to FVIII, induce the formation of FXa via interaction with FIXa on an activated platelet, and support the formation of a blood clot. The activity can be assessed *in vitro* by techniques well known in the art such as e.g. clot analysis, endogenous thrombin potential analysis, etc. Factor VIII molecules according to the present invention have FVIII activity being at least about 10%, at least 20%, at least 30%, at least 40%, at least 50%, at least 60%, at least 70%, at least 80%, at least 90%, and 100% or even more than 100% of that of native human FVIII.

### Albumin binder conjugates

It is known that the *in vivo* properties of such proteins can be improved by the use of albumin binding side chains. Such side chains, or "albumin binders", can be attached to the protein prior to administration and can, for example, stabilise the protein *in vivo* or improve or extend the *in vivo* half-life of the protein.

In the present invention we describe attachment of protractor groups to proteins. When conjugated to a protein, such groups may extend the in vivo circulation half-life of the protein compared to the un-conjugated protein. These protractor groups are of the type we name as "albumin binders" and include derivatives of fatty acids. These groups may or may not have affinity for albumin. The attachment of albumin-binders to proteins or peptides has been shown to potentially increase the plasma half life of said proteins or peptides. A class of typical albumin binders are derived from fatty acids, because albumin is capable to bind highly hydrophobic molecules. Therefore, compounds having a -(CH₂)₁₂-moiety are possible albumin binders in the context of this invention. If such a compound is attached to a protein or peptide and results in an increased plasma half life of said protein or peptide, it is understood that the albumin binder may contribute to the overall increase of plasma half life by either binding to albumin and/or by other mechanisms.

The albumin binder may thereby promote the circulation of the derivative with the blood stream.

In one embodiment, the albumin binder-protein conjugate is a molecule in which a single albumin binder has been attached to the protein. In other embodiments, more than one albumin binder has been attached to the protein, preferably two, three, four, or five.

The albumin binder (albumin binding moiety) may comprise a portion which is particularly relevant for the protraction of circulation in the blood stream, which portion may accordingly be referred to as a protracting moiety. The protracting moiety may or may not have affinity for albumin. The protracting moiety is preferably at, or near, the opposite end of the albumin binding moiety as compared to its point of attachment to the peptide.

In a preferred embodiment, the albumin binder is, or comprises, a side chain that is capable of forming non-covalent complexes with albumin. The albumin binder may bind albumin non-covalently and/or reversibly. The albumin binder may bind albumin specifically. As is clear from the methods described below, the albumin binder may bind to cyclodextrin. The albumin binder may bind cyclodextrin non-covalently and/or reversibly. The albumin binder may bind cyclodextrin specifically.

An albumin binder as described herein is generally a hydrophobic group.

The other portion of the albumin binding moiety, i.e. the portion in-between the protracting moiety and the point of attachment to the peptide, may be referred to as a linker moiety, linker, spacer, or the like. However, the presence of such a linker is optional, and hence the albumin binding moiety may be identical to the protracting moiety.

In particular embodiments, the albumin binding moiety and/or the protracting moiety is lipophilic, and/or negatively charged at physiological pH (7.4).

The albumin binding moiety and/or the protracting moiety may be covalently attached to an amino group of the peptide by conjugation chemistry such as by alkylation, acylation, or amide formation; or to a hydroxyl group, such as by esterification, alkylation, oximation.

In a preferred embodiment, an active ester of the albumin binding moiety and/or the protracting moiety is covalently linked to an amino group of a sialic acid residue or a sialic acid derivative, under formation of an amide bond.

For the present purposes, the terms "albumin binding moiety", "protracting moiety", and "linker" include the un-reacted as well as the reacted forms of these molecules. Whether or not one or the other form is meant is clear from the context in which the term is used.

The albumin binding moiety may be, or may comprise a fatty acid or fatty diacid or a derivative or either thereof.

The term "fatty acid" refers to aliphatic monocarboxylic acids having from 4 to 28 carbon atoms, such as 16 carbon atoms. It is preferably unbranched, and/or even numbered, and it may be saturated or unsaturated.

The term "fatty diacid" refers to fatty acids as defined above but with an additional carboxylic acid group in the omega position. Thus, fatty diacids are dicarboxylic acids.

The nomenclature is as is usual in the art, for example -COOH, as well as HOOC-, refers to carboxy; -C₆H₄- to phenylen; -CO-, as well as -OC-, to carbonyl (O=C<); and C₆H₅-O- to phenoxy.

In a preferred embodiment, the albumin binding moiety of the present invention comprises a fatty acyl group (-(CH₂)ₙ-CO-, where n= 1,2,3,...40) or an omega-carboxy fatty acyl group (HO₂C-(CH₂)ₙ-CO-, where n= 1,2,3,...40) linked the peptide or protein via a linker and a sialic acid residue or sialic acid derivative.

In a preferred embodiment the linker moiety, if present, has from 2 to 80+ C-atoms, preferably from 5 to 70 C-atoms. In additional preferred embodiments, the linker moiety, if present, has from 4 to 20 hetero atoms, preferably from 2 to 40 hetero atoms, more preferably from 3 to 30 hetero atoms. Particularly preferred examples of hetero atoms are N-, and O-atoms. H-atoms are not hetero atoms.

In another embodiment, the linker comprises at least one OEG molecule, and/or at least one glutamic acid residue, or rather the corresponding radicals (OEG designates 8-amino-3,6-dioxaoctanic acid, i.e. this radical: -NH-(CH₂)₂-O-(CH₂)₂-O-CH₂-CO-).

In one preferred embodiment, the linker moiety comprises a di-carboxyl residue linked to a sialic acid residue by an amide bond. In preferred examples, the di-carboxyl residue has from 2-30 C-atoms, preferably 4-20 C-atoms, more preferably 4-10 C-atoms. In additional preferred examples, the di-carboxyl residue has from 0- 10 hetero-atoms, preferably 0- 5 hetero-atoms.

Examples of albumin binders in accordance with the present invention are illustrated in Figure 1C and Figure 11C.

In another preferred example, the linker moiety comprises a group containing both an amino and a distal carboxyl-group linked to a sialic acid residue by an amide bond through its distal carboxyl groups. In one preferred embodiment this group is an OEG group.

In a particular preferred embodiment, the sialic acid is linked to an N-linked or O-linked glycan of the protein.

The amino acid glutamic acid (Glu) comprises two carboxylic acid groups. Its gamma-carboxy group is preferably used for forming an amide bond with an amino group of a sialic acid residue or a sialic acid derivative, or with an amino group of an OEG molecule, if present, or with the amino group of another Glu residue, if present. The amino group of Glu in turn forms an amide bond with the carboxy group of the protracting moiety, or with the carboxy group of an OEG molecule, if present, or with the gamma-carboxy group of another Glu, if present. This way of inclusion of Glu is occasionally briefly referred to as "gamma-Glu".

### Separation methods

The present invention relates to methods that allow the purification of protein-albumin binder conjugates. In particular, the methods described herein may be used to achieve separation between such conjugates and the unconjugated parent protein or to achieve purification of such conjugates from a mixture comprising the conjugates and the parent protein.

When preparing such albumin binder conjugates, it is highly desirable to be able to be able to remove as much as possible of the unmodified form of the protein. If significant amounts of unmodified protein remain in the protein formulation that is used, the specific activity of the formulation can appear to be deceptively high. This specific activity reflects the total specific activity of the protein in the formulation, not just the specific activity of the conjugated protein. Since the conjugated protein may have different *in vivo* characteristics to the unmodified protein, such "false positives" caused by unmodified protein in the formulation can give unreliable information about the efficacy of the final formulation. In particular, where a conjugated form of protein has significantly improved stability *in vivo* when compared with the unconjugated protein, the presence of unconjugated protein in a formulation may result in the formulation showing a high specific activity, even if it does not have the same effectiveness *in vivo* as a formulation comprising only conjugated protein.

The methods described herein thus allow for the purification of conjugated protein from unconjugated protein, thereby excluding such false positives and enabling a more reliable measure of specific activity for a given formulation of conjugated protein.

The conjugates used in the methods described herein may be any of the conjugates described herein. They may comprise any of the proteins discussed herein, particularly those proteins discussed above. They may also comprise any of the albumin binders or albumin binding moieties discussed herein.

The present methods use an affinity chromatography approach.

The basic conditions throughout the present methods may be selected by the skilled person based on routine methods and known purification techniques in use for the protein of interest. For example, a suitable chromatography column, chromatography resin and buffer system may be selected for a given protein of interest. Preferably the conditions selected are such that the protein of interest, or the protein-conjugate of interest, remain in an active form throughout the chromatography process. The use of strong buffers is generally to be avoided. As mentioned above, many existing separation methods require the presence of components that can have a detrimental effect on the protein being purified, or that can lead to the degradation of the solid support. The present invention avoids these disadvantages.

Preferably, the buffers used in the present methods do not comprise any components that adversely affect the activity or function of the protein being purified. For example, the buffers used in the present methods preferably do not comprise isopropanol, or comprise isopropanol only in low concentrations such as less than 30%, less, than 20%, less than 10% or less than 5%.

Preferably the buffers used in the present methods are selected to allow re-use of the solid support after the final elution step. For example, buffer components should be avoided which will degrade or reduce the efficiency of the solid support. For example, the buffers used in the present methods preferably do not comprise octanoic acid.

The sample of interest, i.e. a sample comprising the conjugates to be purified, is applied to a solid support under suitable conditions to allow the binding of the conjugates to the solid support. The sample of interest is one which comprises or which may comprise a mixture of (a) a protein that is conjugated to an albumin binder as described herein and (b) said protein in a form that is not conjugated to an albumin binder. For example, the sample of interest may be a composition resulting from a process for attaching an albumin binder moiety to a protein. It may therefore comprise successfully formed protein-albumin binder conjugates and also protein molecules to which the albumin binder has not been attached.

The solid support may be any suitable support for use in affinity chromatography. For example, a chromatography column may be utilised. A chromatography resin, such as a resin based on cross-linked agarose, e.g. a Sepharose resin, may be utilised.

The present invention utilises such a standard chromatography system, but additionally allows for binding of the conjugated protein to the solid support, e.g. the chromatography resin.

The solid support comprises a substance that allows for such binding of the conjugates. In particular, the solid support comprises a substance that is capable of binding to the albumin binder in the conjugate, wherein said substance is selected from the group consisting of albumin and cyclodextrin.

The substance may be present as a component part of the solid support, such as a component of a chromatography resin. The substance may be immobilised on the solid support, for example the substance may be attached to the surface of the solid support. Examples 1 and 2 describe suitable methods that may be used to immobilise a substance, such as cyclodextrin or albumin, on a chromatography column.

A suitable substance for use in or on a solid support may be any substance that binds an albumin binder as described herein. Preferably the substance binds to an albumin binder non-covalently and reversibly. Preferably, the substance selectively binds to the albumin binding moiety as described herein. By selective binding is meant that the substance binds to the albumin binder in preference to other molecules. For example, the substance may bind to the albumin binder but may not bind to another molecule, or may bind to the other molecule with lesser affinity. The substance may bind to more than one type of albumin binder as described herein, but may not bind to another molecule, or may bind to the other molecule with lesser affinity. The substance may bind to an albumin binder but may not bind a protein molecule, or may bind to a protein with lesser affinity. In particular, the substance may bind to the albumin binder when the albumin binder is conjugated to a protein as described herein, but may not bind, or may bind with lesser affinity to the unmodified (unconjugated) form of the same protein that does not comprise an albumin binder.

In one embodiment, the substance for use in or on a solid support may be albumin.
In one embodiment, the substance may be cyclodextrin. The cyclodextrin may be of the beta- and/or alpha- type. The cyclodextrin may also be of a modified type, such as hydroxypropyl cyclodextrin. The solid support used in the methods described herein may thus comprise albumin and/or cyclodextrin. The solid support used in the methods described herein may have albumin and/or cyclodextrin immobilised thereon.

The methods of the present invention therefore comprise a step of contacting a mixture comprising protein and conjugated protein as described herein with a suitable solid support, under conditions that allow for the binding of the conjugated protein to the solid support. Preferably the conjugated protein is bound to the solid support in preference to the unconjugated protein. This may be achieved by, for example, incorporating albumin and/or cyclodextrin in the solid support or immobilising albumin and/or cyclodextrin in or on the solid support.

This step thus achieves binding of the conjugated form of protein to the solid support. It will be appreciated that the specific conditions of this loading step, such as other buffer components, buffer volume and flow rate, may be optimised for a given form of conjugated protein. The key feature of this loading step is that the conjugated protein is loaded onto, and binds, the solid support.

Preferably, this loading step is followed by one or more wash steps. These wash steps are intended to remove unbound protein, and in particular to remove unbound unconjugated protein, from the solid support. Again, the specific conditions such as buffer components, buffer volume and flow rate may be adjusted to each particular method. The buffer used for washing may be the same as the buffer used for loading. Preferably a wash buffer, buffer volume and flow rate are selected which do not result in elution of the bound conjugated protein during the course of the wash step(s).

The eluent resulting from this washing may optionally be monitored to identify any protein components that are removed. For example, the eluent may be divided into fractions and samples of each fractions analysed to assess their composition or to identify and/or quantify particular components of interest. It may be preferred to stop this washing procedure at a suitable time to minimise the loss of the wanted product, i.e. the conjugated protein, from the solid support. This can be achieved by monitoring for the presence of the conjugated protein in the eluent and stopping the wash when the conjugated protein starts to be seen. It may be preferred to stop this washing at a suitable time to remove the maximum amount of unconjugated protein from the column. This can be achieved by monitoring the removal of unconjugated protein in the eluent and stopping the wash only when unconjugated protein is no longer being removed. Multiple wash steps may be utilised.

After the solid support has been washed, the conjugated protein bound to the solid support is eluted.

The elution comprises contacting the solid support with the substance capable of binding to the albumin binding moiety.

In one embodiment, this elution may be achieved using the same or a similar buffer to that used in the loading and/or wash steps. For example, a single wash and elution process may be used. This approach may be appropriate when the same buffer that is used to wash unbound protein from the column will also eventually remove the bound protein. For example, different fractions of eluent may be obtained, with the earlier fractions comprising a higher proportion of unbound (unconjugated) protein and the later fractions comprising a higher proportion of the bound (conjugated) protein. Suitable fractions may thus be selected to provide a suitable purified form of the conjugated protein, with a reduced content of unconjugated protein.

Such an approach may be of particular use where the conjugated protein binds relatively weakly to the solid support. A suitable elution buffer can thus be selected so as to allow the gradual separation of the conjugated protein from the solid support. Because the conjugated protein will be bound to the solid support and the unconjugated protein will not be bound to the solid support, such a method may still achieve separation and/or purification of these components.

An alternative approach may utilise a particular component in the elution buffer to achieve elution of the conjugated protein. For example, once the wash step(s) described above are completed, a suitable separation of conjugated and unconjugated protein may have been achieved and it may simply be desired to remove the product of interest from the solid support. This can be achieved by including in the elution buffer a component that competes with the substance in the solid support for binding by the conjugated protein. For example, the elution buffer may comprise a component that competitively binds the albumin binding moiety. The addition of such a component to the elution buffer will allow the disruption of the binding between the product and the solid support, thus releasing the product into the eluant.

This approach may be used wherein the albumin binder binds weakly to the solid support or wherein the albumin binder binds strongly to the solid support. In particular, this elution approach may be used wherein the albumin binder will not be removed from the solid support by continuation of the washing step(s). This approach may be preferred where it is desired to achieve a high degree of separation between the conjugated and unconjugated forms of protein. That is, where the albumin binder binds strongly to the solid support, extensive or stringent washing may be performed in order to remove the maximum amount of unbound components from the solid support. In this embodiment, such extensive washing should not remove the bound conjugated protein from the solid support. When removal of unbound components is complete, for example when the eluant from the wash step no longer comprises any protein, then elution of the bound conjugated protein may be achieved by adding a suitable elution component to the elution buffer.

A suitable elution component may be any substance capable of competitively binding the albumin binding moiety, as defined in the claims. For example, the elution buffer may comprise cyclodextrin. The cyclodextrin may of the beta- and/or alpha- type. The cyclodextrin may also be of a modified type, such as hydroxypropyl cyclodextrin. The cyclodextrin may thus interact with the albumin binding moiety, thus weakening the interaction between that moiety and the solid support and releasing it into the eluant.

A suitable concentration of cyclodextrin for elution may be, for example, up to 100 mM, such as between 1 mM and 100 mM, between 1 mM and 50 mM, between 5 mM and 20 mM. A suitable concentration of cyclodextrin and the elution buffer may be about 10 mM.

The methods described above may be utilised to improve the purity of protein that is conjugated to an albumin binder and/or to reduce the amount of native, unconjugated protein from a composition of such protein-albumin binder conjugates.

Accordingly, the present invention relates to a method for purification of a protein that is conjugated to an albumin binding moiety from a mixture comprising (i) said protein in said conjugated form and (ii) said protein in a form that is not conjugated to said albumin-binding moiety, the method comprising:
(a) providing a solid support comprising a substance capable of specifically binding to the albumin binding moiety, wherein said substance is selected from the group consisting of albumin and cyclodextrin;
(b) contacting said solid support of (a) with said mixture comprising protein and conjugated protein under suitable conditions for binding of the albumin binding moiety to the substance as defined in (a); and
(c) eluting components bound to the solid support, wherein said elution comprises contacting the solid support with the substance capable of binding to the albumin binding moiety.

In accordance with these methods:
- the protein may be any protein that is discussed herein;
- the albumin binding moiety may be any albumin binder or any albumin binding moiety as described herein;
- the solid support and substance used in step (a) may be any support or substance discussed above in the context of solid supports;
- step (b) represents a loading step and may be carried out as discussed above;
- one or more washing steps as described above may be included between step (b) and step (c);
- said elution step (c) may be carried out as described above, for example by including an additional elution component such as cyclodextrin in the elution buffer.

The steps described above may be carried out one or more times. For example, a purified composition prepared in accordance with the methods described herein may be subjected to additional purification steps. A purified composition prepared in accordance with the methods described herein may be further purified by repeating the loading and elution steps described above (steps (b) and (c)).

The affinity chromatography methods described herein also allow for the solid support to be re-used. Many chromatorgraphy methods lead to degradation or a change in properties of the solid support, meaning that such solid supports have a limited life-span or require regeneration between uses. Because the solid supports used in the present methods are not altered or degraded by the purification methods, they can be readily reused without the need for regeneration and with a longer life-span than other chromatography solid supports.

The present invention thus relates to a method for purification of a protein that is conjugated to an albumin binding moiety from a mixture comprising (i) said protein in said conjugated form and (ii) said protein in a form that is not conjugated to said albumin-binding moiety, the method comprising:
(a) providing a solid support comprising a substance capable of specifically binding to the albumin binding moiety, wherein said substance is selected from the group consisting of albumin and cyclodextrin;
(b) contacting said solid support of (a) with said mixture comprising protein and conjugated protein under suitable conditions for binding of the albumin binding moiety to the substance as defined in (a); and
(c) eluting components bound to the solid support.

Said elution of step (c) comprises contacting the solid support with the substance capable of binding to the albumin binding moiety.
In a second embodiment, said substance is capable of competitively binding to the albumin binding moiety.
In a third embodiment, said substance is cyclodextrin.
In a fourth embodiment, said substance is applied to said solid support in a gradient of increasing concentration in step (c).
In a fifth embodiment, the substance capable of specifically binding to the albumin binding moiety in step (a) is albumin or cyclodextrin.
In a sixth embodiment the protein is selected from Factor VIII, growth hormone, Factor VII, GLP-1, insulin, or a variant form of any thereof.
In a seventh embodiment, the albumin binding moiety extends the half life of the protein *in vivo.*
In an eighth embodiment the albumin binding moiety comprises a fatty acid or fatty acid derivative.
In another embodiment, the fatty acid derivative comprises a fatty diacid with at least 12 methylene units.
In another embodiment, the fatty acid or fatty acid derivative is linked to a coagulation factor via a sialic acid of an N-linked glycan.
In another embodiment, the coagulation protein is factor VII or factor VIIa.
In another embodiment, the coagulation protein is factor VIII.
In another embodiment, the coagulation protein is factor IX
In a final embodiment, the albumin binding moiety is as defined in Figure 1C or Figure 11C.

### Abbreviations:

ABz: aminobenzoic acid or aminobenzoyl
Boc : *tert* butyloxycarbonyl
CD: Cyclodextrin
CMP : cytidine monophosphate
CV : column volumes
DCM : dichloromethane, CH₂Cl₂, methylenechloride
DIC : diisopropylcarbdiimide
DIPEA : N,N-diisopropylethylamine
DMF : N,N-dimethylformamide
DMSO : dimethylsulfoxide
FLD : fluorescence detection
Fmoc : 9H-fluoren-9-ylmethoxycarbonyl
GSC: Glycyl sialic acid CMP ester
HC: heavy chain^{··}
HOAt: 7-Azahydroxybenzotriazole
HOBt: Hydroxybenzotriazole
HPCD: Hydroxypropyl β-cyclodextrin
HPLC : high pressure liquid chromatography
Lac : lactosyl or lactose
LC: light chain
LC-MS : liquid chromatography - mass spectrometry
Lys(Mtt)-OH : (S)-6-[(Diphenyl-p-tolyl-methyl)-amino]-2-amino-hexanoic acid m/z : mass to charge ratio
MBP: mannose binding protein
MQ : MilliQ water (highly purified water)
MS : mass spectrometry
NAN : N-acetyl neuraminic acid
NMP : N-methylpyrrolidin-2-one
NMR : nuclear magnetic resonance spectroscopy
OEG : (2[2-(amino)ethoxy]ethoxy)acetic acid
O-*t*-Bu : *tert* butyl ester
PSC: polyethylene glycol sialic acid CMP ester
RP : reversed phase
rt or RT : room temperature
SC: single chain (HC and LC are covalently connected) *t*-Bu : *tert* butyl
TFA : trifluoroacetic acid
THF : tetrahydrofuran
Thx : *trans*-4-aminomethylcyclohexancarboxylic acid
TIPS : triisopropylsilane
UM : 4-Methylumbelliferyl
wt: wild type
Amino acid abbreviations follow IUPAC conventions.
Buffer abbreviations follow Stoll, V. S. and Blanchard, J. S., Methods of Enzymology, 182, 1990, Academic Press, 24-38.

### EXAMPLES

### Example 1. Preparation of a cyclodextrin column

A sepharose column with immobilised beta-CD was prepared from Sigma product M2314, 6-monodeoxy-6-monoamino-beta-cyclodextrin and an NHS Hitrap column (GE Healthcare). The Hitrap column contained 10 µmol reactive groups. 5 µmol of amino-CD M2314 (MW 1171, 5.9 mg) was dissolved in 1 ml coupling buffer.

| | | |
|---|---|---|
| A: | 0.2 M NaHCO₃, 0.5 M NaCl, pH 8.3 | (Coupling buffer) |
| B: | 0.5 M ethanolamine, 0.5 M NaCl, pH 8.3 | (de-activation buffer) |
| C: | 0.1 M NaOAc, 0.5 M NaCl, pH 4.0 | (washing buffer) |
| D: | 2 M glycine-HCl, pH 2.0 | (acidification buffer) |

The coupling was done strictly according to GE Healthcare instructions protocol 71-7006-00 AT for HiTrap columns. In brief, 1 ml of the amino-CD solution was used for the coupling which was carried out for 40 min at room temperature. Coupling yield was not determined. The column was then repeatedly de-activated (buffer B)/washed (buffer C) according to the protocol.

### Example 2. Preparation of an HSA column

A sepharose-HSA column was prepared using a NHS HiTrap 1 ml column as example 1. The substitution was 10 µmol/ml. HSA from Sigma (A1653) was used. Ca. 1.5 ml of a 10 mg/ml (0.15 umol/ml) solution was prepared in buffer A and filtered through 0.45 µm filter.

| | | |
|---|---|---|
| A: | 0.2 M NaHCO₃, 0.5 M NaCl, pH 8.3 | (Coupling buffer) |
| B: | 0.5 M ethanolamine, 0.5 M NaCl, pH 8.3 | (de-activation buffer) |
| C: | 0.1 M NaOAc, 0.5 M NaCl, pH 4.0 | (washing buffer) |
| D: | 2 M glycine-HCl, pH 2.0 | (acidification buffer) |

The coupling was done strictly according to GE Healthcare instructions protocol 71-7006-00 AT for HiTrap columns. 1 ml of the HSA solution was used. Coupling yield was determined according to the "acidification to pH 2.5" method:

After coupling (in buffer A), the column was washed with buffer A and 3 ml wash-out was collected. 50 µl of the solution was diluted with 50 µl buffer D. i.e. wash-out was diluted x 6. The original coupling solution 50 µl was diluted with 50 µl buffer D, then 10 x with buffer A:buffer D 1:1, i.e. total dilution: x 20. A280 was measured for the two solutions in triplicate:

| Sample | A280 | Mean | Un-diluted |
|---|---|---|---|
| Coupling solution x 20 diluted | 0.225 | 0.225 | 4.50 |
| | 0.244 | | |
| | 0.207 | | |
| Wash-out x 6 diluted | 0.228 | 0.215 | 1.29 |
| | 0.221 | | |
| | 0.197 | | |

| | | | |
|---|---|---|---|
| Coupling efficiency: (4.50-1.29)/4.5=71% | | | |

The column was then repeatedly de-activated (buffer B)/washed (buffer C) according to the protocol. Then, the buffer was changed to HEPES 50 mM, pH 7.5. Washing for ca. 45 min. It was finally washed with imidazole 20 mM, pH 7.3, 10 mM CaCl₂, 150 mM NaCl for ca. 30 min.

### Example 3. Preparation of a reference column, with no affinity ligand attached.

A reference column was prepared by following the protocol set out in Example 1 except that the coupling step was exchanged with a washing with 6 ml de-activating buffer + reaction for 30 min.

### Example 4. Separation of hGH and albumin binder hGH conjugate on the cyclodextrin column of Example 1.

An albumin binder hGH conjugate was prepared having a structure as shown in Figure 1C and purified by means of HPLC using the following procedure:

The preparation is outlined in the scheme below. It consisted of two steps:
Step 1: Preparation of an albumin binder containing an aniline handle for conjugation to an hGH-aldehyde
Step 2: Conjugation to the hGH-aldehyde. The aldehyde-function is located at the Gln-141 residue of hGH.

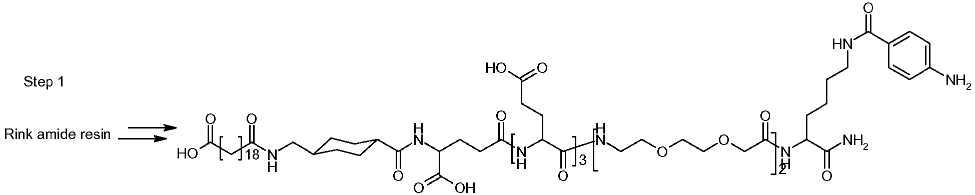

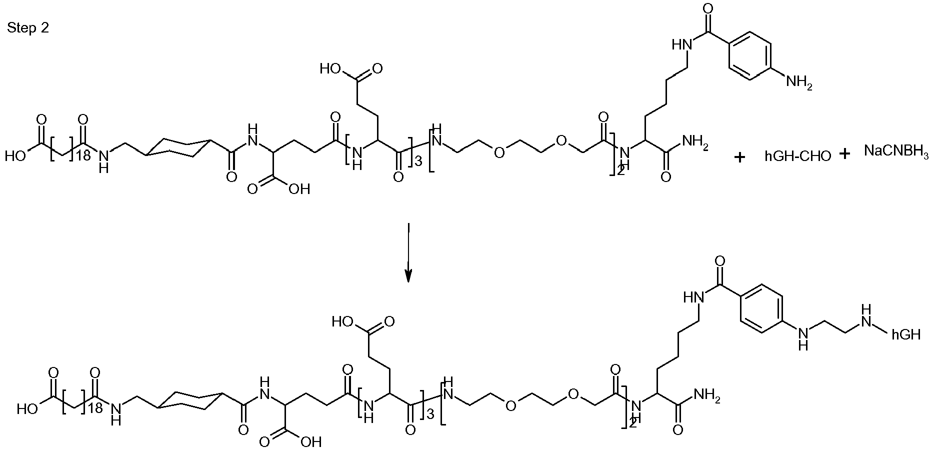

### Step 1

Fmoc-protected Rink-amide resin (2.0 g, 0.6 mmol/g) was weighed out in a reaction flask and swelled in 3x30 ml NMP. The resin was drained and treated twice with 30 ml 25% piperidine in NMP, for 10 min and 1 hour respectively. The resin was drained and washed with 6x30ml NMP.

Next, Fmoc-Lys(Mtt)-OH (1500 mg, 2 eq.) and HOBt (324 mg, 2.4 eq.) were weighed out and dissolved in 20 ml 0.5 mM bromophenol blue in NMP. This solution was added to the drained resin followed by the addition of DIC (374 µl, 2.4 eq.). The reaction mixture was shaken at ambient temperature for 3.5 hours until colour change from dark blue to light green. Finally, the resin was drained and washed with 6x30ml NMP, 3x30ml DCM, and drained.

The resin was treated with 10 ml hexafluoroisopropanol for 10 minutes (shaken). It was drained, and washed with 3x30ml DCM, and hexafluorisopropanol treatment and washing were repeated. Finally, the resin was drained and washed with 3x30ml NMP.

Boc-4-ABz-OH (569 mg, 2.4 eq.) and HOBt (324 mg, 2.4 eq.) were weighed out and dissolved in 20 ml 0.5 mM bromophenol blue in NMP. This solution was added to the drained resin followed by the addition of DIC (374 µl, 2.4 eq.). The reaction mixture was shaken at ambient temperature overnight until colour change from dark blue to light green. The resin was drained and washed with 6x30ml NMP. A small amount of beads was taken to test cleavage with TFA showing the desired product bound to the resin.

The resin was drained and treated twice with 30 ml 25% piperidine in NMP, for 10 min and 1 hour respectively. The resin was drained, washed with 6x30ml NMP, and drained leaving the wet resin 10 g (wet). An amount of 1.7 g (0.2 mmol) of the wet resin was withdrawn for further reaction.

Coupling of Fmoc-OEG-OH was performed using the following procedure: Fmoc-OEG-OH (154 mg, 2 eq. compared to the resin loading) and HOBt (135 mg, 2 eq.) were weighed out and dissolved in 5 ml 0.5 mM bromophenol blue in NMP. This solution was added to the drained resin followed by the addition of DIC (62 µl, 2 eq.). The reaction mixture was shaken at ambient temperature overnight until colour change from dark blue to light green. Finally, the resin was drained, washed with 6x5 ml NMP, and drained. The resin was drained and treated twice with 5 ml 25% piperidine in NMP, for 10 min and 1 hour respectively. The resin was drained and washed with 6x5 ml NMP. Next, the coupling of Fmoc-OEG-OH (coupling time 3.5 hours) and removal of the Fmoc group was repeated so as to obtain a compound with two terminal OEG-groups and a free amine.

In the following five steps, the amino acids Fmoc-Glu(O-t-Bu)-OH (three couplings), Fmoc-Glu-OtBu (one coupling) and Fmoc-tranexamic acid were coupled and Fmoc-deprotected by the same procedure as for Fmoc-OEG-OH described above, with the following amounts and conditions (HOBt: 135 mg, 2 eq. DIC: 62 µl, 2 eq. were used for all couplings):
1st coupling: Fmoc-Glu(OtBu)-OH: 170 mg, 2 eq.,coupling time: overnight
2nd coupling: Fmoc-Glu(OtBu)-OH: 170 mg, 2 eq., coupling time: 2 h
3rd coupling: Fmoc-Glu(OtBu)-OH: 170 mg, 2 eq., coupling time: overnight
4th coupling: Fmoc-Glu-OtBu: 170 mg, 2 eq., coupling time: overnight
5th coupling: Fmoc-Thx-OH: 152 mg, 2 eq., coupling time: overnight
Finally, the Fmoc-group was removed as described above.

Dodecanedioic acid mono-tBu ester (HO₂C-(CH2)₁₈-COOtBu, 159 mg, 2 eq., see WO 2005/012347 A2) and HOBt (135 mg, 2 eq.) were weighed out and dissolved in 5 ml 0.5 mM bromophenol blue in NMP. This solution was added to the drained resin followed by the addition of DIC (62 µl, 2 eq.). Coupling time was overnight. Finally, the resin was washed with 6x5ml NMP, 6x5ml DCM and drained.

The resin was treated with 5 ml TFA:water:DCM:TIPS 6.3 /0.3/2.9/0.4 (V/V) for 1 h (shaken) and filtered into 50 ml cold diethyl ether. The preciptate generated was isolated by centrifugation, washed 3 times with diethyl ether, and dried at rt overnight leaving the crude product as a solid, 160 mg.

The crude solid was dissolved in a mixture of TFA, water, acetonitrile and DMSO and purified by preparative RP-HPLC using a gradient from 40-80% acetonitrile. The pure compound was isolated as a solid, 70 mg, and was identified by LC-MS.

### Step 2

The following solutions were prepared
Solution 1: N^{ε141}-(2-hydroxy-3-aminopropyl) hGH (200 mg, 9 µmol, in 150ml buffer, see WO2005070468A2) dissolved in buffer 20 mM triethanolamine, 0.12 M NaCl.
Solution 2: The compound isolated in step 1 (30 mg, 2eq.) dissolved in 3.6 ml AcOH and 2.4 ml water.
Solution 3: 3-methylthio-1-propanol :290 mg dissolved in 4 ml water
Solution 4: NaIO₄ (48mg) dissolved in 1ml water.
Solution 5: NaCNBH₃ (22 mg dissolved in 500 µl water + 15 ul AcOH): 2 eq., 20 µmol, 30 µl was used

Solution 1 was wrapped in Alu-foil, followed by addition of solution 3 (4.4 ml) and solution 4 (333 µl). The reaction mixture was incubated in the refrigerator for 40 minutes. The reaction mixture was buffer exchanged to 50 mM MES, pH 6.0 using an Amicon Ultracel 10 k ultrafiltration device (4 times centrifugation (4000 rpm/min, 10°C 10-20 minutes). The final volume was 6 ml. To this solution was added solution 2. The reaction mixture was gently shaken at ambient temperature for 30 minuttes after which solution 5 (30 µl) was added. The reaction was allowed to continue overnight.

After 20 hours of reaction time, a conversion of ca. 60% to the desired hGH-conjugate was observed by HPLC. The reaction mixture was diluted with 20 ml water and filtered. The reaction mixture was buffer exchanged to water using an Amicon Ultracel 10 k ultrafiltration device (4 times centrifugation (4000 rpm/min, 10°C, 15 minutes). The final volume was 10 ml. This solution was slowly added to 30 ml 20% triethanolamine, pH 8.5. The final pH of the solution was 8.5. This solution was then buffer exchanged to 20 mM triethanolamine, pH 8.5 using an Amicon Ultracel 10k ultrafiltration device (4 times centrifugation (4000 rpm/min, 10°C, 15 minutes). The final volume was 47 ml.

This solution was applied to a Mono-Q 10/100 column (GE Healthcare) and eluted using 20 mM triethanolamine, pH 8.5 as starting buffer (A) and 20 mM triethanolamine, pH 8.5, 1M NaCl as elution buffer (B). Fractions containing the desired compound eluted around 400 mM B-buffer and were pooled. These pooled fractions were buffer exchanged and re-purified using the same chromatographic system. Fractions containing the desired product were buffer exchanged to 10 mM ammonium bicarbonate using an Amicon Ultracel 10k ultrafiltration device (4 times centrifugation (4000 rpm/min, 10°C, 15 minutes). The final volume was 13.5 ml. Finally, the solution was freeze dried yielding the desired product as a white solid 14.7 mg. The product termed 005 was identified by LCMS.

### Chromatographic experiment:

0.5 mg of hGH was mixed with 0.5mg of the albumin binder hGH conjugate 005.
The mixture was subjected to chromatography on the column described in Example 1 using the following conditions:

| | |
|---|---|
| Flow: | 0.5 ml/min |
| A-buffer: | 20 mM imidazole, 10 mM CaCl₂, 150 mM NaCl, pH 7.3, 10 % glycerol |
| B-buffer: | A + 20 mM hydroxypropyl cyclodextrin |
| Temp: | 22°C |

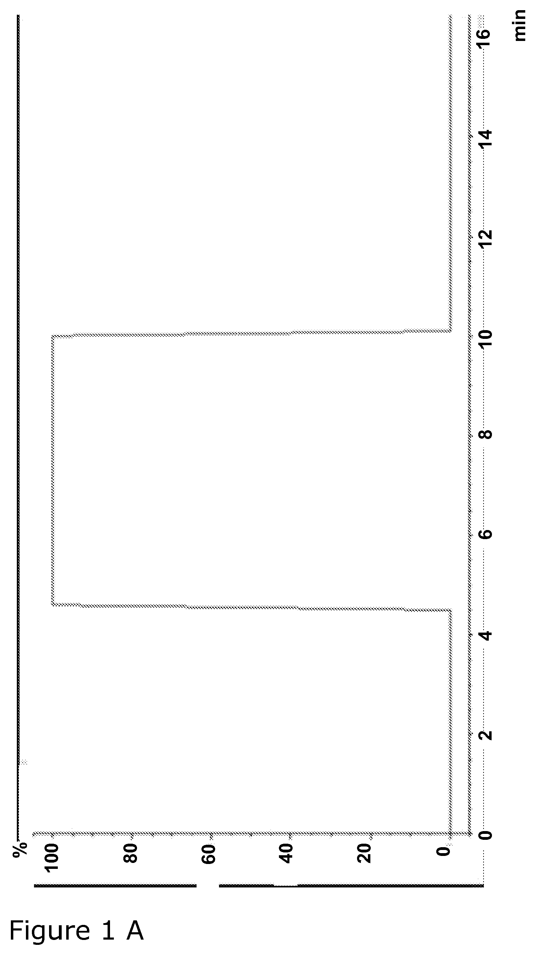

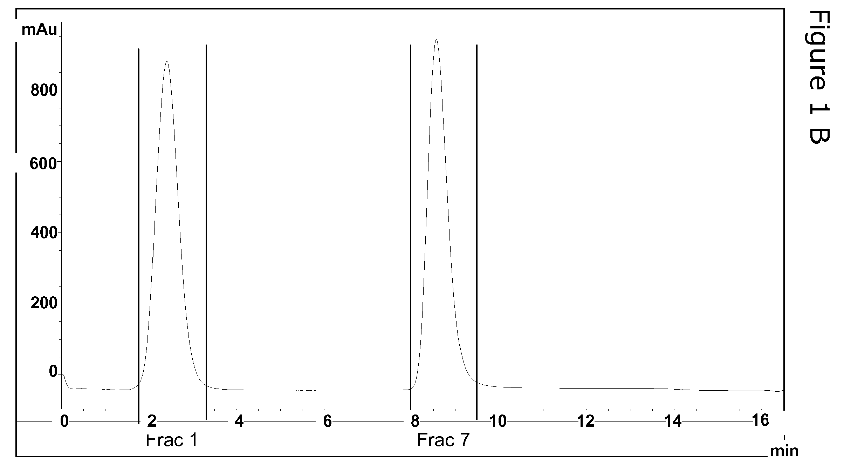

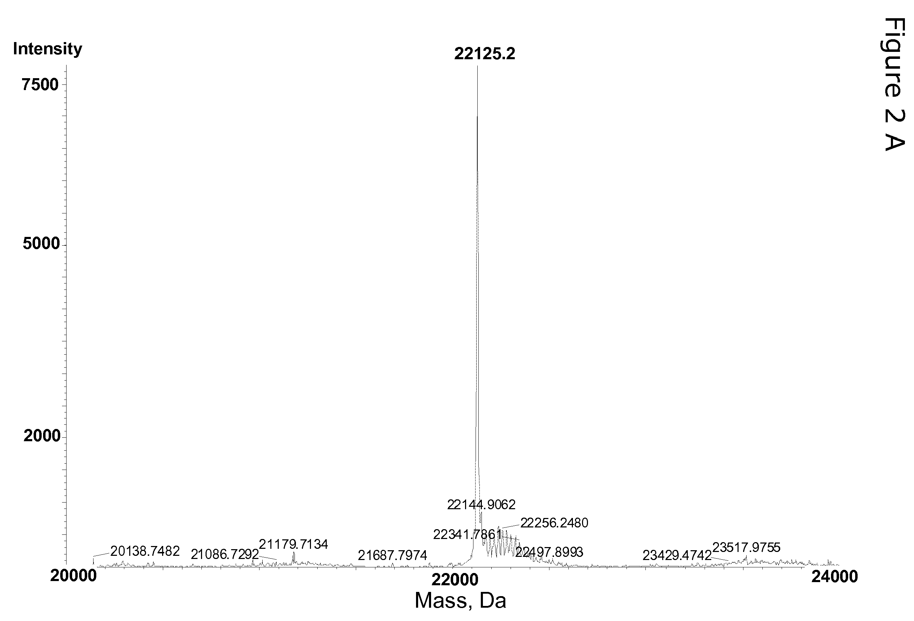

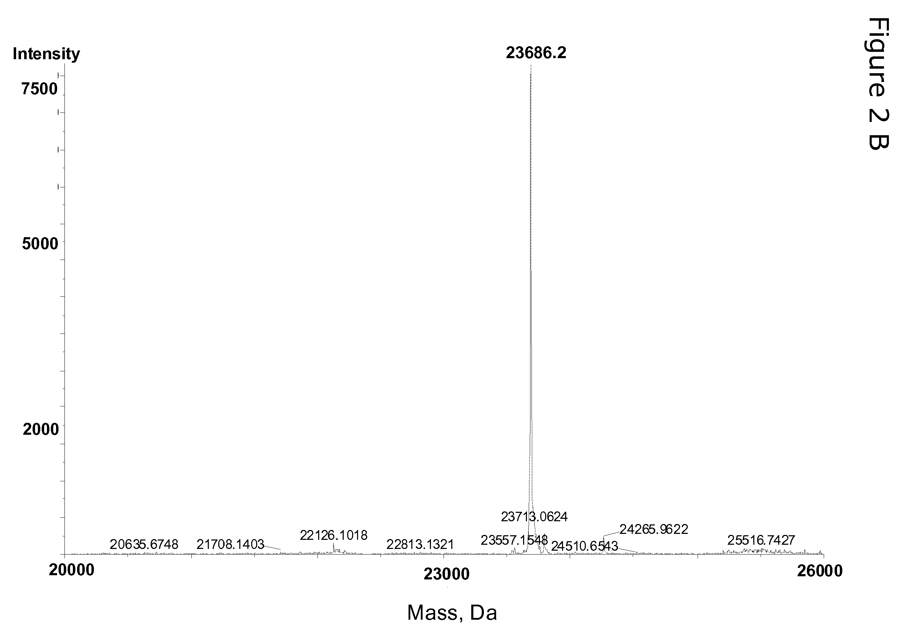

Figure 1A shows the elution gradient profile and Figure 1B shows the chromatogram that was obtained. All chromatograms are FLD signals (ext. 280, emm. 348). Figure 2 shows the mass spectrometry profiles for fraction 1 (Figure 2A) and fraction 7 (Figure 2B). Faction 1 was identified as unmodified hGH and fraction 7 was identified as albumin binder-hGH conjugate.

This example shows that hGH and an hGH-albumin binder conjugate can be separated on a column with immobilised cyclodextrin.

### Example 5. Separation of hGH and albumin binder hGH conjugate on the reference column of Example 3.

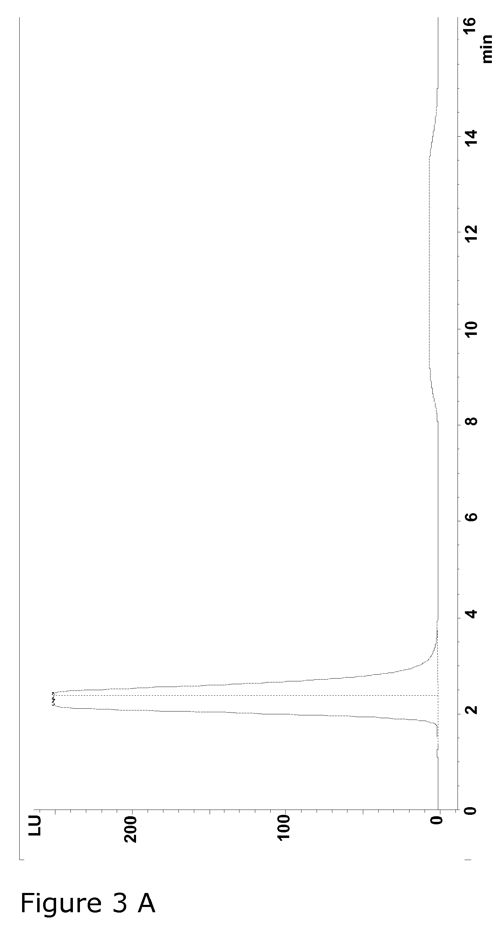

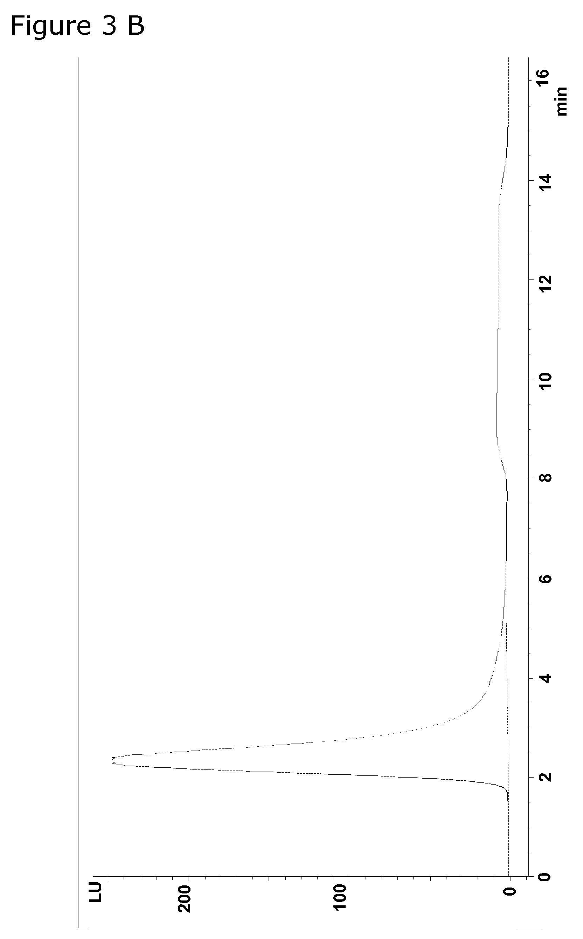

Conditions were as set out in Example 4. No separation between hGH and hGH-albumin binder conjugate was observed (see Figure 3). This demonstrated that the affinity interaction in the cyclodextrin column is specific. All chromatograms are FLD signals

### Example 6. Separation of hGH and albumin binder hGH conjugate on the albumin column of Example 2.

The hGH and albumin binder hGH conjugate were processed using the column described in Example 2.

| | |
|---|---|
| Sample hGH: | 2 mg/ml in 20 mM HEPES pH 7.5 |
| Sample | Mixture of hGH / hGH-albumin binder conjugate as of example 4, 2 mg/ml in 20 mM HEPES pH 7.5 |
| Flow: | 0.5 ml/min |
| A-buffer: | 20 mM imidazole, 10 mM CaCl₂, 150 mM NaCl, pH 7.3 |
| B-buffer: | 35% i-PrOH in A |
| Temp: | 25°C |

Figure 4 shows the resultant chromatograms. All chromatograms are FLD signals Cyclodextrin was then included in the B buffer as follows:

| | |
|---|---|
| A: | 20 mM imidazole, 10 mM CaCl₂, 150 mM NaCl, pH 7.3 |
| B: | 10 or 20 mM HPCD in A |
| Temp: | 22°C |

Figure 5 shows the resultant chromatograms. All chromatograms are FLD signals

This example shows that hGH and an hGH-albumin binder conjugate can be separated on the column with immobilised HSA of Example 2, however, the separation efficiency of this column is much poorer than the immobilised CD-column of Example 1.

### Example 7. Separation of FVIII and albumin binder FVIII conjugate, on the albumin column of Example 2.

Factor VIII and an albumin binder FVIII conjugate prepared in accordance with Example 11 were processed using the column described in Example 2.

The Factor VIII molecule used in this example (N8) was a B-domain deleted Factor VIII protein (Thim et al. Haemophilia (2010) 16, 349). The albumin binder used in the FVIII-conjugate in this experiment was as shown in Figure 11C.

| | |
|---|---|
| Flow: | 0.5 ml/min |
| A-buffer: | 20 mM imidazole, 10 mM CaCl₂, 150 mM NaCl, pH 7.3, 10 % glycerol |
| B-buffer: | 20 mM HPCD in A |
| Temp: | 22°C |

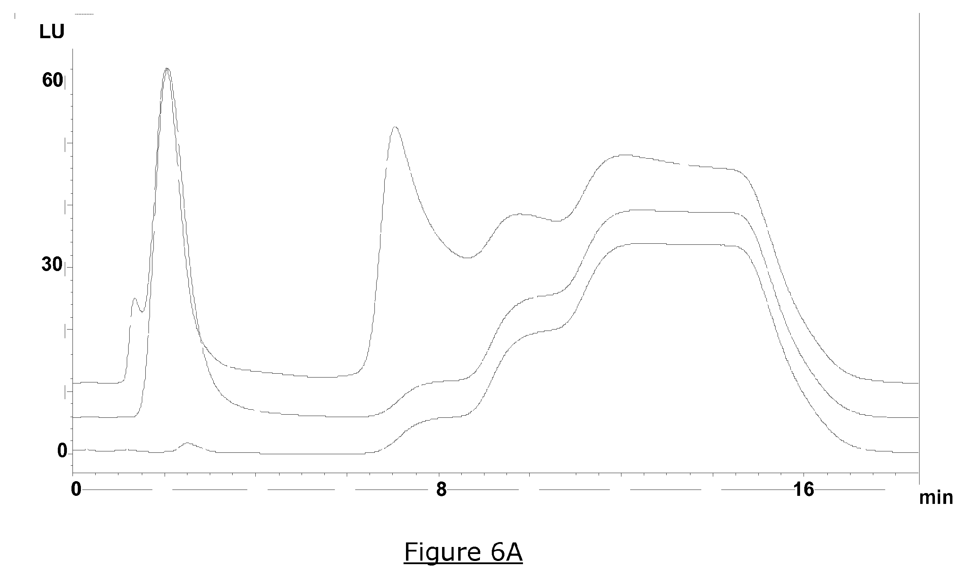

A step gradient was used, jumping from 0% to 20% to 60% to 100% of B buffer. Figure 6A shows the resultant chromatogram. All chromatograms are FLD signals. The crude reaction mixture was found to contain a compound that was more retained on the column compared to FVIII.

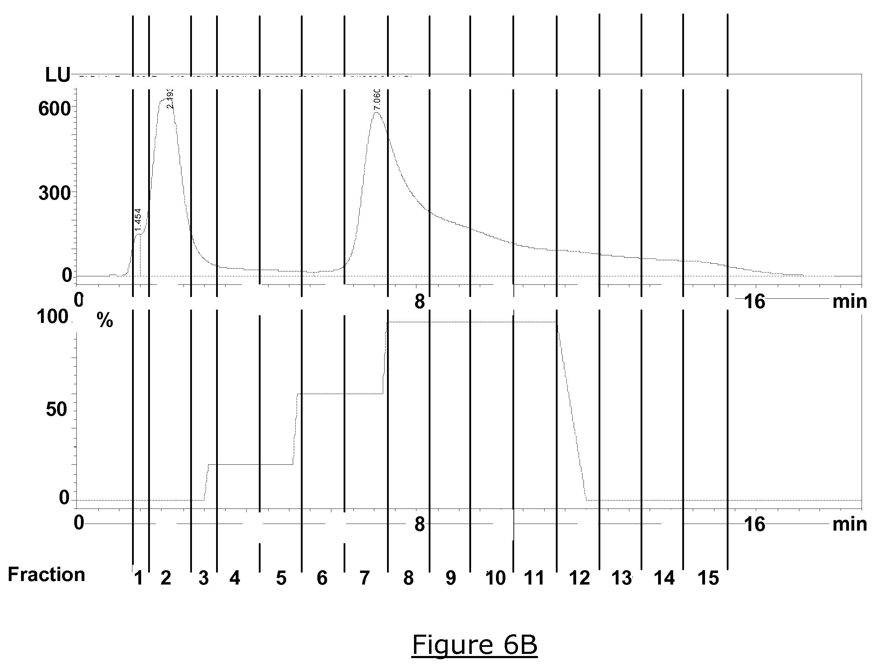

Figure 6B shows a chromatogram resulting from use of 30 ul of FVIII -albumin binder conjugate, crude reaction mixture, injected (ca. 60 ug protein).

SDS PAGE was carried out on the fractions shown in Figure 6B. 20 µl were taken from each fraction. For N8 and 152-1:, 20 µl of solution was diluted x 150. An appropriate amount of thrombin was added to all samples. These were incubated for 10 min at 37°C then 7.5 µl LDS buffer was added.

| | |
|---|---|
| Procedure: | Invitrogen protocol |
| Gel: | Nupage 7 % Tris-Acetate Gel, 1.0 * 15 wells |
| Buffer: | NuPage Tris-Acetate SDS Running buffer (20 x). 40 ml diluted with 760 ml MQ-water |
| Sample buffer: | Nupage LDS Sample Buffer (4x) |
| Marker: | HiMark HMW standard |
| Program: | 70 min, 150 V, 120 mA & 25 W |
| Volume: | 7 µl / well. |
| Staining kit: | SilverQuest (without fixing) |

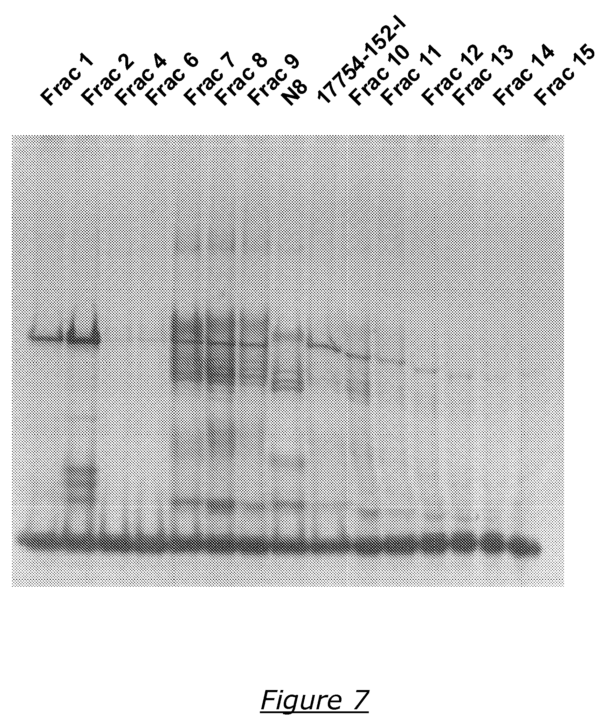

The results are shown in Figure 7. The FVIII-albumin binder conjugate was found to be bound to the column, and could be eluted again with buffer containing HPCD

### Example 8. Separation of FVIII and albumin binder FVIII conjugate, on the cyclodextrin column of Example 1.

The same conditions as example 4 were used. 158-1 and 158-11 represent two preparations in which FVIII has been partially modified with an albumin binder.

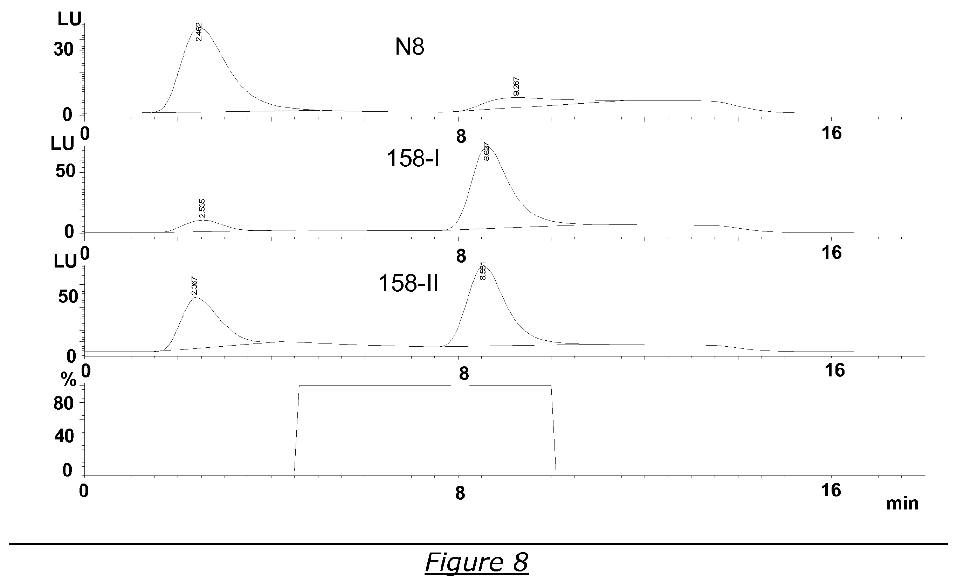

The results are shown in Figure 8. It can be seen from these chromatograms that the two preparations display different degrees of modification.

### Example 9. Separation of FVIII and albumin binder FVIII conjugate (partially converted, crude reaction mixture) on the cyclodextrin column of Example 1.

The reagents used were as follows:

| **Reagent** | **Amount** | **Amount** | **Concentration** | **eq** |
|---|---|---|---|---|
| **Factor VIII** | 0.25 mg | 1.44 nmol | 5 mg/ml | 1 |
| **Sialidase, * 0.6m U/µl** | 26 mU/12.5 µl | 6.5 mU | 0.6->1.8 U/ml gel | |
| **MBP-ST3Gal3** | 25 µl | 22 mU | 0.9 mg/ml / 1.06 U/mg | |
| **Substrate 1 Albumin binder GSC** | 15 µl | 15 nMol | 1 nmol/µl | 10 |

0.25mg N8 (internal batch, in 50 µl) was thawed. Immobilised sialidase was washed with 3x50 µl H₂O, then 3x50 µl HEPES-buffer. This was spinned down and excess buffer was removed. MBP-ST3Gal-III was thawed. Substrate 1 was thawed.

To the washed sialidase was added: N8 (50 µl); MBP-ST3Gal-III (25 (µl); Substrate 1 (15 µl). The reaction was incubated at 32°C for a period of 1 h. 5 µl was withdrawn for HPLC (CD-column) analysis and diluted up to 50 µl with buffer A. 50 µl was injected.

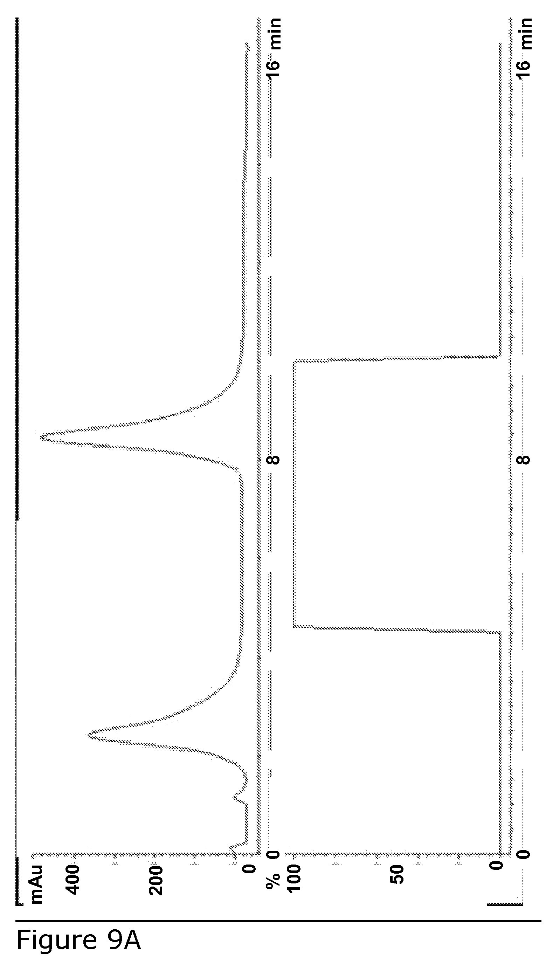

Figure 9A shows the analytical run.

| | |
|---|---|
| Flow: | 0.5 ml/min |
| Buffer A: | 20 mM imidazole, 10 mM CaCl₂, 150 mM NaCl, pH 7.3, 10 % glycerol |
| Buffer B: | A + 20 mM HPCD |
| FLD: | ext 280, emm 348 (for F8) |
| Temp: | 22°C |

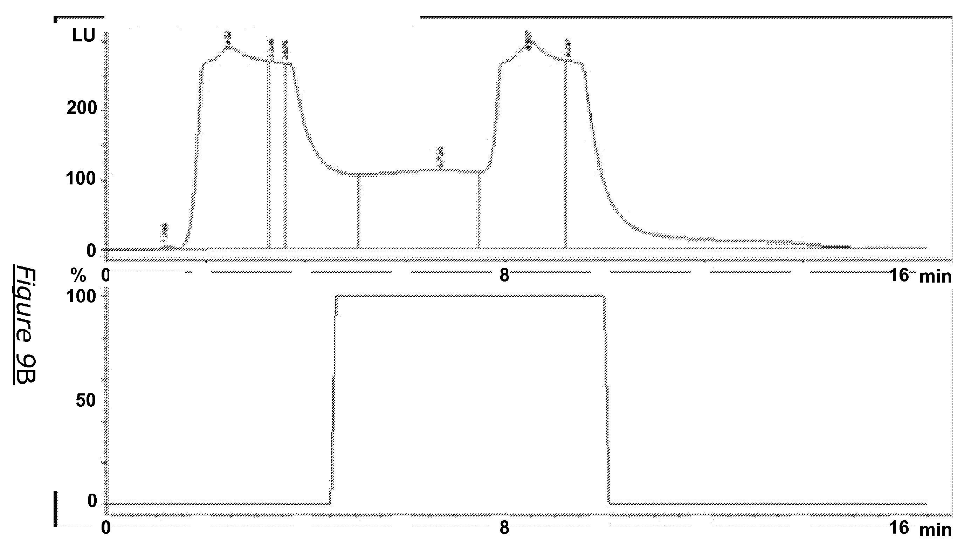

The reaction mixture was filtered, using Pierce Spin column. Vol ca 85 µl. The complete mixture was applied to the CD column, method as above. Figure 9B shows the preparative run

The following fractions were collected manually.

| | | | |
|---|---|---|---|
| Fraction 3: | 2.0 -> 2.5min. | Fraction 4: | 2.6-> 3.0min. |
| Fraction 5: | 3.1 -> 4.1min. | Fraction 6: | 4.2-> 5.2min. |
| Fraction 7: | 5.3-> 6.3min. | Fraction 8: | 6.4-> 7.4min. |
| Fraction 9: | 7.5-> 8.0min. | Fraction 10: | 8.1-> 9.0min. |
| Fraction 11: | 9.1-> 9.4min. | Fraction 12: | 9.5 -> 10.0min. |
| Fraction 13: | 10.1 -> 10.6min. | Fraction 14: | 10.7 -> 11.2min |
| Fraction 15: | 11.3-> 12.0min. | Fraction 16: | 12.1-> |

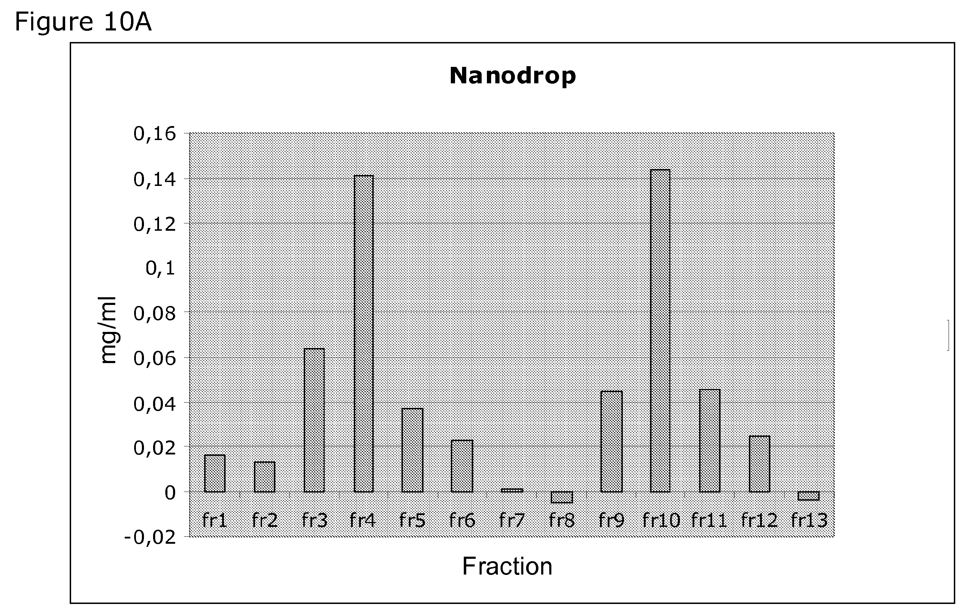

Figure 10A shows the protein content in each fractions as measured by Nanodrop (A280, E1% 14.6)

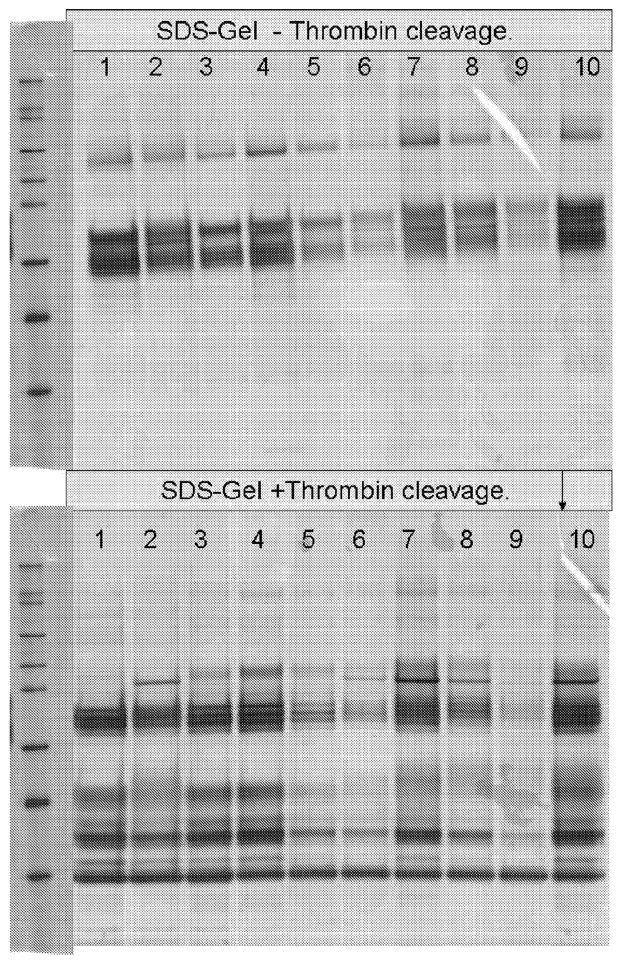

SDS PAGE gel showed that unmodified N8 (fractions 3-5) had been separated from albumin binder N8 conjugate (fractions 9-12), eg. by comparing fractions 5 and 11 (slightly shifted towards higher mass in the thrombin cleaved samples). See Figure 10B

### Example 10. Mono-functionalisation of wt B-domain deleted FVIII O-glycan using ST3Gal-I and isolation of the product using a CD-column

wt B-domain deleted FVIII (5.7 mg/ml, 352 µl, 10. 2 nmol) in buffer (20 mM imidazole, 10 mM CaCl₂, 0.02 %Tween 80, 1 M glycerol, pH 7.3, 500 mM NaCl) was mixed with sialidase (*A*. *Urifaciens*, 12 µl, 0.43 mg/ml, 130 U/ml, ref.: Christensen and Egebjerg, Biotechnol. Appl. Biochem., 41, 225-231), HIS-ST3Gal-I (21.6 U/mg, 100 (µl) and substrate 1 (150 µl, 200 nmol). Substrate 1 is shown in Figure 11C.

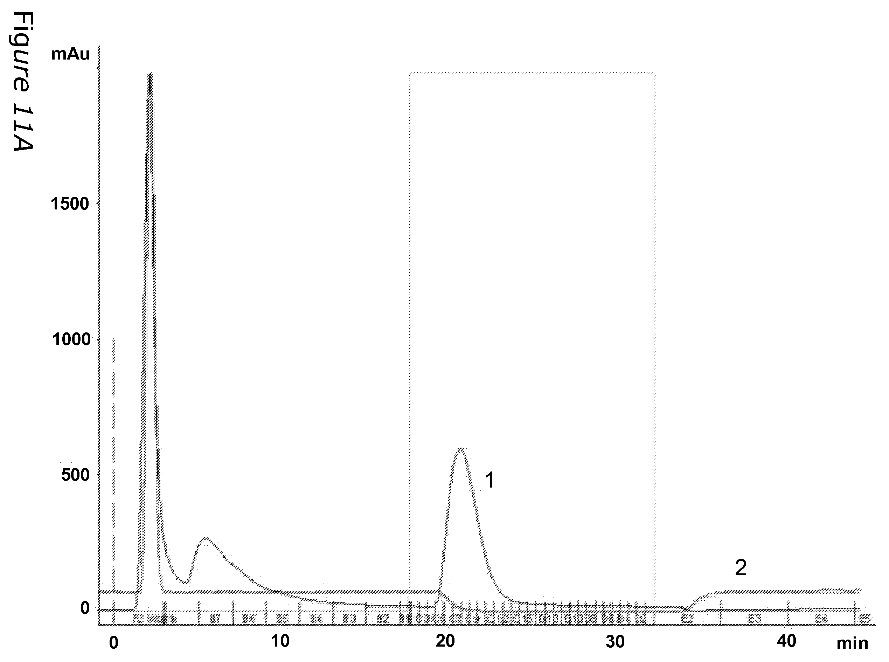

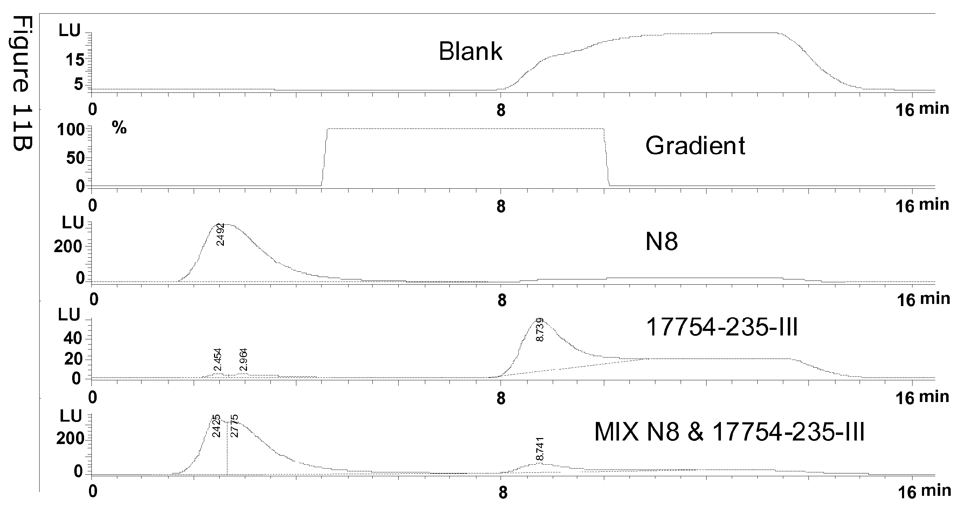

The mixture was incubated at 32C for 2.5 h after which the conjugated FVIII was isolated by cyclodextrin-affinity chromatography: The material was loaded to the immobilised cyclodextrin column and washed with 18 ml of starting buffer 20 mM imidazole, 10 mM CaCl₂, 150 mM NaCl, pH 7.3, 10 % glycerol. The elution was performed as a step to 100 % elution buffer 20 mM imidazole, 10 mM CaCl₂, 150 mM NaCl, pH 7.3, 10 % glycerol, 20 mM hydroxypropylcyclodextrin, whereby the product eluted as 730 µg in 2.5 ml (see figure 11). The product was then treated with CMP-NAN (40 µl, 34 mg/ml) and MBP-SBD-ST3Gal-III (200 µl, 0.33 mg/ml, ca. 0.5 U/ml) at 32°C for 1 h, and frozen at - 80°C.

After thawing, the product was purified by spin column AIEC (Vivapure Q Mini M, strong anion exchange) which had been equilibrated with 10 ml 20 mM imidazole, 10 mM CaCl₂, 0.02 %Tween 80, 1 M glycerol, pH 7.3. Then, the column was washed with
1. 2x10 ml of 20 mM imidazole, 10 mM CaCl₂, 0.02 %Tween 80, 1 M glycerol, pH 7.3,
2. 2x10 ml of 20 mM imidazole, 10 mM CaCl₂, 0.02 %Tween 80, 1 M glycerol, pH 7.3,50mM NaCl
3. 2x10 ml of 20 mM imidazole, 10 mM CaCl₂, 0.02 %Tween 80, 1 M glycerol, pH 7.3, 200 mM NaCl.

Finally, the FVIII conjugate was eluted with 20 mM imidazole, 10 mM CaCl₂, 0.02 %Tween 80, 1 M glycerol, pH 7.3, 1 M NaCl, giving the conjugate 586 µg in 700 ul. It was applied to a Superdex 200 10/300 GL gelfiltration column and eluted with histidine (1.5 mg/ml), CaCl₂ (0.25 mg/ml), Tween 80 (0.1 mg/ml), NaCl (18 mg/ml), sucrose (3 mg/ml).

The eluate contained one major product eluting 0.58 column volumes which was collected and contained 348 µg of the desired conjugate in 2.5 ml. The product was characterised by non-reduced SDS PAGE indicating that the bands corresponding to A1 and A3C1C2 containing the modified glycans were shifted towards higher MW. Also, analytical cyclodextrin-affinity chromatography with and without wt B-domain deleted FVIII proved that the conjugate (termed 17754-235-III) bound to cyclodextrin whereas wt B-domain deleted FVIII did not (see Figure 11B)

### Example 11. Coupling of substrate 1 to the N-glycans of wt B-domain deleted FVIII using ST3Gal-III

Asialo wt B-domain deleted FVIII was obtained by subjecting to a PEGylation reaction using sialidase (*A*. *Urifaciens*), ST3Gal-I, and 40 kDa PSC as described in WO2009/108806 A1. After completion of the reaction, the (un-PEGylated) asialo-FVIII was separated from the PEGylated FVIII by AIEC. Finally, the asialo-FVIII was buffer-exchanged using a MonoQ AIEC column. An amount of 37.4 mg of the asialo-FVIII isolated from the first AIEC in 82 ml buffer was diluted with MQ water (150 ml) to obtain a condutivity of 12 mS/cm and was loaded to the column which had been equilibrated with buffer histidine (1.5 mg/ml), CaCl₂ (0.25 mg/ml), Tween 80 (0.1 mg/ml), NaCl (50 mM), sucrose (3 mg/ml). The product was eluted using a gradient of buffer histidine (1.5 mg/ml), CaCl₂ (0.25 mg/ml), Tween 80 (0.1 mg/ml), NaCl (1 M), sucrose (3 mg/ml). The product eluted at ca. 500 mM NaCl.

Asialo wt B-domain deleted FVIII obtained as above (1 mg, 5.6 nmol, in 800 ul buffer histidine (1.5 mg/ml), CaCl₂ (0.25 mg/ml), Tween 80 (0.1 mg/ml), NaCl (500 mM), sucrose (3 mg/ml) was treated with ST3Gal-III (500 µl, conc. to 50 µl, 500 mU) and substrate 1 (45 µl, 60 nmol). The reaction was incubated at 32°C for 21.5 h (crude product).

A sample was withdrawn for SDS PAGE analysis. Then, a solution of CMP-NAN (2 mg, 3.1 µmol in 20 µl buffer (20 mM imidazole, 10 mM CaCl₂, 0.02% Tween 80, 500 mM NaCl, 1 M glycerol, pH 7.3)) was added and incubated at 32°C for 1 h.

The reaction mixture was diluted to 19 ml with ca 18 ml 20 mM imidazole, 10 mM CaCl₂, 0.02 %Tween 80, 1 M glycerol, pH 7.3. The salt concentration was then ca. 28 mM. The product was then purified by spin column AIEC as outlined in example 10. The conjugate (820 µg) was recovered in 1 ml. It was applied to a Superdex 200 10/300 GL gelfiltration column and eluted with buffer histidine (1.5 mg/ml), CaCl₂ (0.25 mg/ml), Tween 80 (0.1 mg/ml), NaCl (18 mg/ml), sucrose (3 mg/ml). The eluate contained one peak at ca. 0.43 column volumes and another peak at ca. 0.58 column volumes. The first peak was identified by SDS PAGE as being ST3Gal-III enzyme (probably in an aggregated form). The second peak contained the desired conjugate, isolated as 273 µg in 2.5 ml. The product was characterised by non-reduced SDS PAGE with prior thrombin cleavage, according to which the bands corresponding to A1 and A3C1C2 containing the modified glycans were clearly shifted towards higher MW.

### Example 12. Characterisation of hGH and hGH albumin binder conjugate on the CD column with different buffer systems

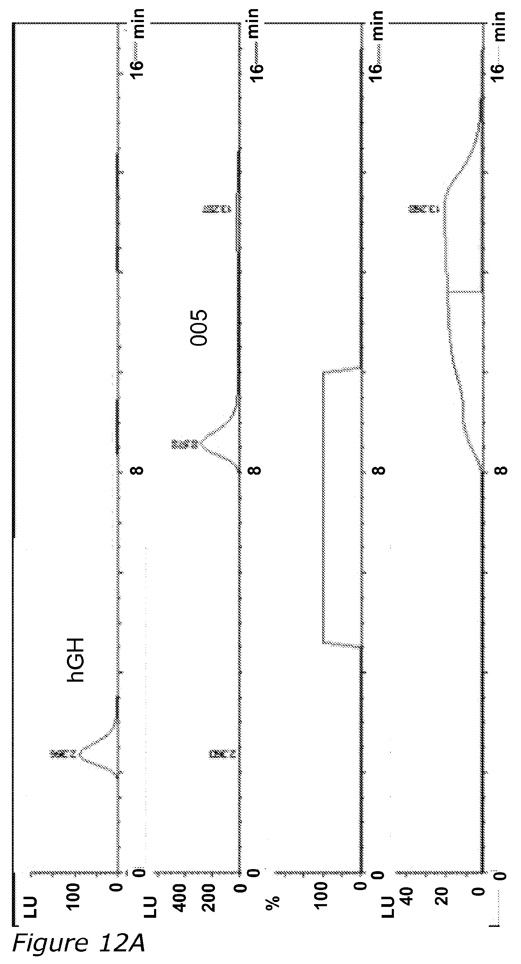

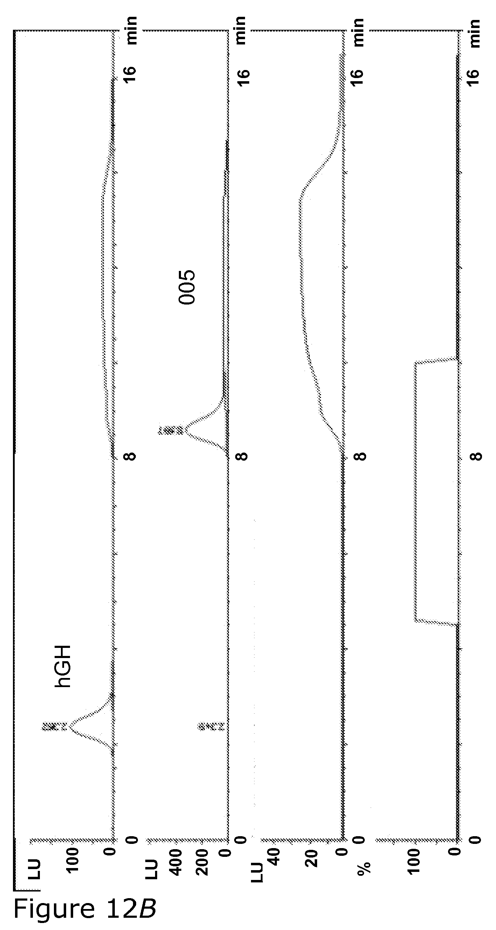

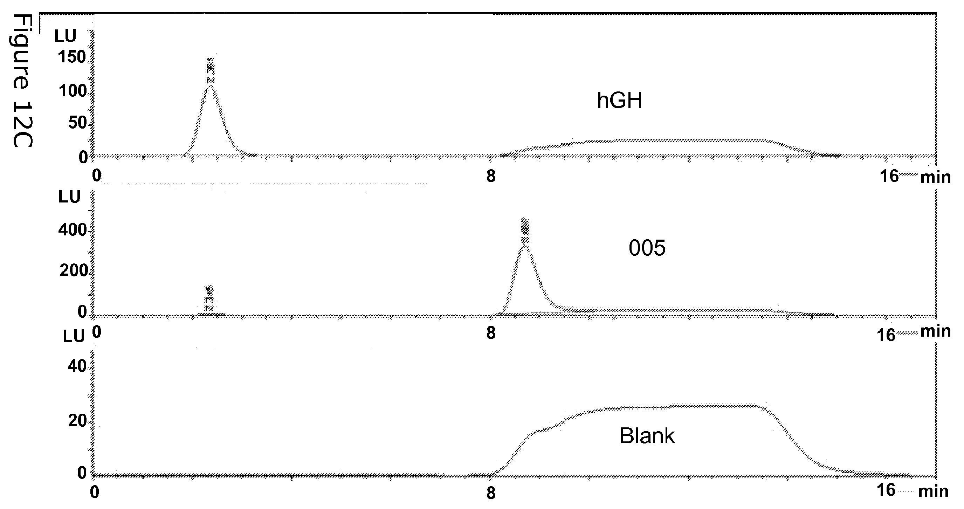

The cyclodextrin column of Example 1 was used. hGH and the hGH-conjugate [005] was injected to the column and eluted with a step gradient as described in example 4. The following buffer systems were investigated:
Buffer system 1 (see Figure 12A):
A: 20 mM imidazole, 10 mM CaCl₂, 150 mM NaCl, pH 7.3, 10 % glycerol
B: 20 mM imidazole, 10 mM CaCl₂, 150 mM NaCl, pH 7.3, 10 % glycerol + 20mM HPCD
Buffer system 2 (see Figure 12B):
A: 20 mM imidazole, 150 mM NaCl, pH 7.3
B: 20 mM imidazole, 150 mM NaCl, pH 7.3 + 20mM HPCD
Buffer system 3 (see Figure 12C):
A: 20 mM Triethanolaminbuffer, pH 7.3
B: 20 mM Triethanolaminbuffer, pH 7.3 + 20mM HPCD

The analytes used were as follows:
hGH: 1.5 mg/0.5 ml. 5 µl was injected.
005: (albumin binding hGH). 0.6mg/0.5ml. 7 µl was injected.

Figure 12 shows that hGH albumin binder conjugate 005 binds to the CD column regardless of buffer system, while hGH itself does not, thus the compounds are separable.

The third chromatogram is a blank sample. The fourth illustrates the step gradient.

### Example 13. Characterisation of different hGH-albumin binder conjugates using the CD column

Buffer system 1 from Example 12 was used for comparing binding of three different hGH-albumin binder conjugates to the CD column of Example 1. Other conditions were the same as in Example 12.

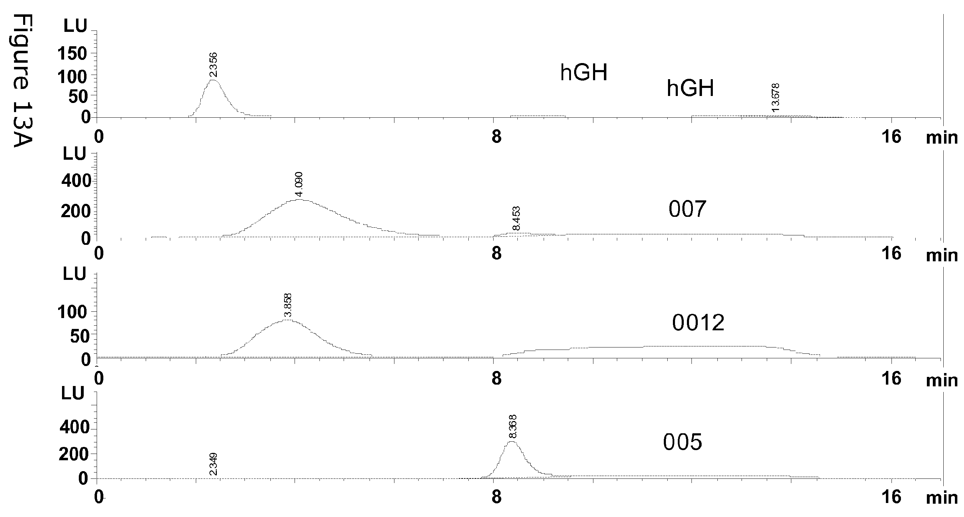

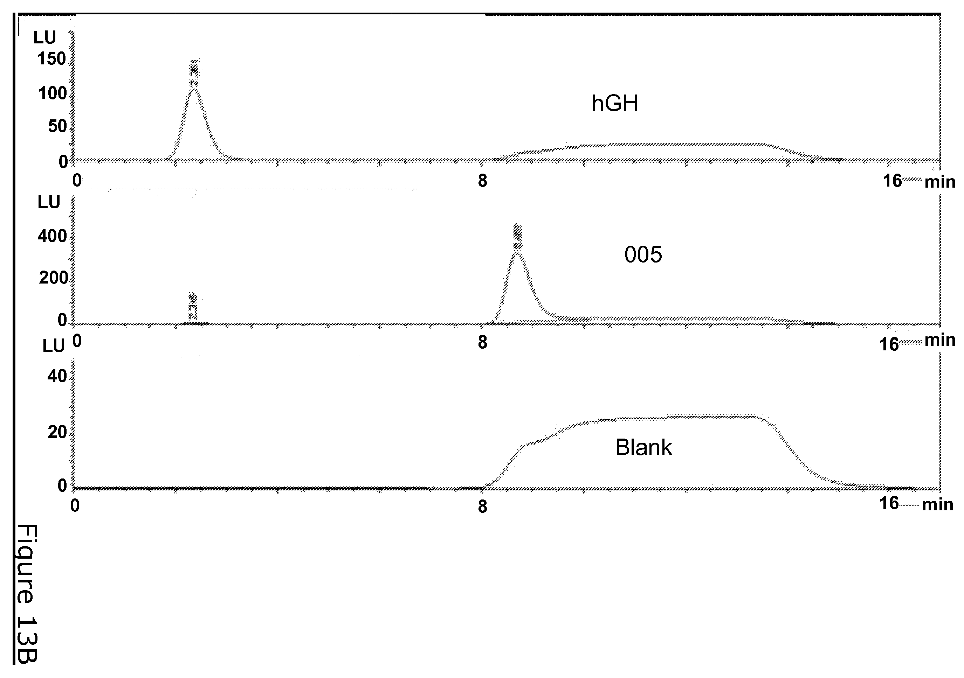

Figure 13A shows that only 005 binds to the CD column, while the hGH-albumin binder conjugates 007 and 0012 are retarded on the column, since the albumin binder-hGH conjugate start eluting before addition of the B-buffer of. Spiking with hGH in the 007 and 0012 samples verified that these conjugates were retarded on the CD column (Figure 13B).

### Example 14. Separation of FVIIa and albumin binder FVIIa conjugate on the cyclodextrin column of Example 1.

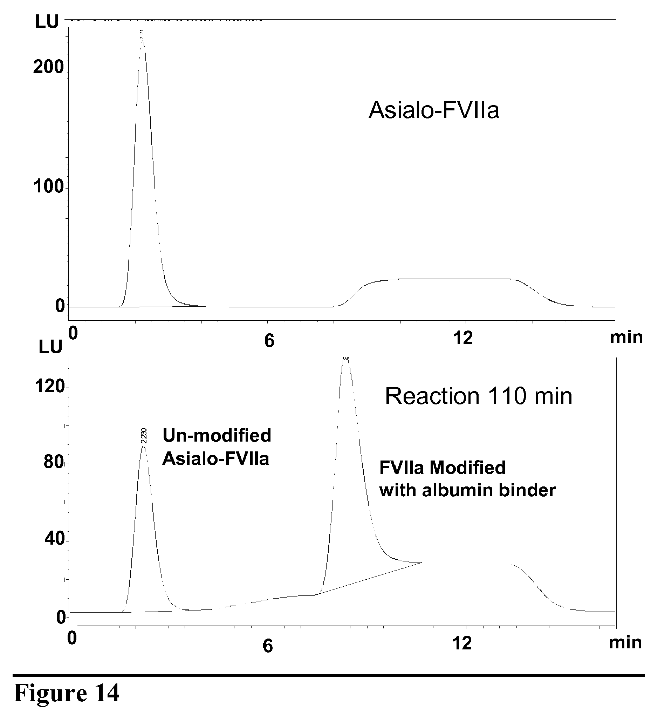

The following reagents were used:
Asialo FVIIa (internal batch): in Gly-Gly buffer, pH 6 (1.39 mg/ml), 500 µl, 13 nmol was used Substrate 1 (albumin binder-GSC): (MW 1869): 2 mg/ml, 30 µl or 32 nmol was used.
MBP-SBP-ST3Gal-III: 1.2 U/ml, 100 µl was used
Asialo-FVIIa referred to a recombinant FVIIa which had been pre-treated with a sialidase and purified. Asialo-FVIIa was thawed, and Substrate 1 and ST3Gal-III were added. The mixture was incubated at 32°C. After 110 min, a sample was separated on the CD-column of Example 1. Chromatographic conditions were as in example 9. Figure 14 shows the result. The first eluting peak corresponded to un-modified asialo-FVIIa and the last eluting peak corresponded to the asialo-FVIIa modified with the albumin binder, as shown by an increase in molecular weight of FVIIa heavy and light chains using SDS-PAGE.

### Example 15. Separation of FIX and albumin binder FIX conjusate on the cyclodextrin column of Example 1.

The following reagents were used:
FIX (internal batch): in 10 mM histidine, 3 mM CaCl₂, 50 mM NaCl buffer pH 6 (11 mg/ml). 100 µl (20 nmol) was diluted to 1 ml with buffer 20 mM imidazole, 10 mM CaCl2, 150 mM NaCl, pH 7.3, 10 % glycerol pH 7.3. 100 µl (2 nmol) was used
Substrate 1 (albumin binder GSC, MW 1869): 2 mg/ml, 7.5 µl (8 nmol) was used MBP-SBP-ST3Gal-III: 1.2 U/ml, 25 µl was used
Sialidase/Neuramidase: 50 µl (6.7 mU) (*C. Perfringens*, type VI-A, N-5254 from Sigma) on agarose (suspension), 0.6-1.8 U/ml gel

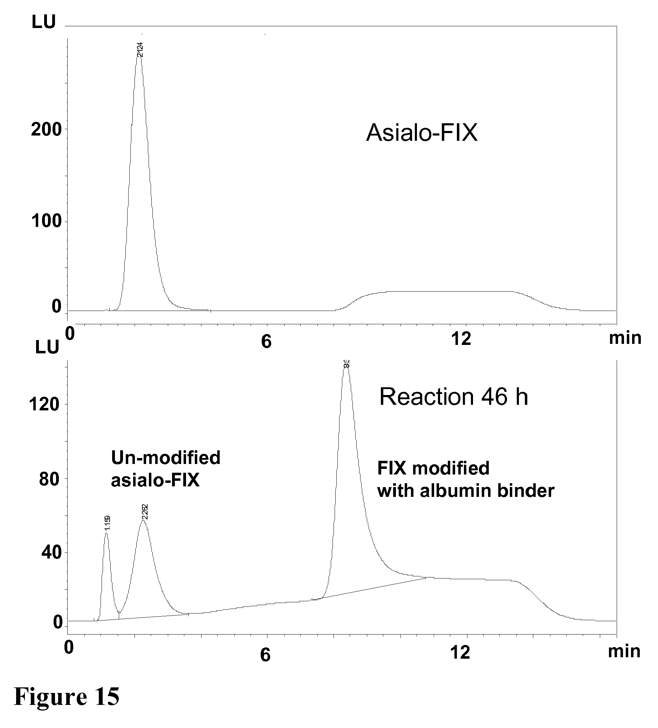

The sialidase was spun down using a Pierce spin column and washed twice with water 500 µl, then three times with buffer 50 mM HEPES, 100 mM NaCl, 10 mM CaCl₂ pH 7. It was spun down and the enzyme was added to the thawed FIX. The reaction was shaken gently at room temperature overnight and then filtered using a Pierce spin column. The resulting filtrate containing asialo FIX was given substrate 1 and ST3Gal-III, and incubated at 32°C for 46 h. A sample of the reaction mixture was separated on the CD-column of Example 1. Chromatographic conditions were as in example 9. Figure 15 shows the result. The first eluting peak corresponds to un-modified asialo-FIX and the last eluting peak corresponds to the asialo-FIX modified with the albumin binder, as shown by an increase in molecular weight of FVIIa heavy and light chains using SDS-PAGE. The gels also showed the presence of small amounts of FIXa generated by self-activation of FIX.

### Example 16. Preparation of albumin binder NHS ester

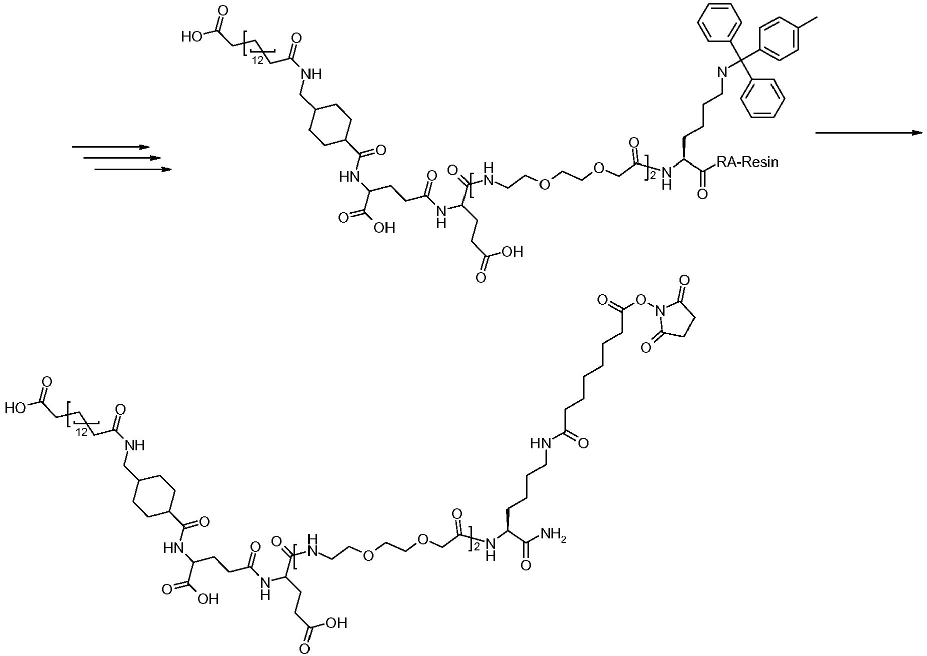

Rink-Amide resin (0.4g, 0.25 mmol) was used for synthesis on a CEM Liberty microwave peptide synthesizer. Standard Fmoc chemistry protocols were used with the following amino acids being used and in that order (all solutions with 7 eq. of amino acid in NMP containing 0.3 M HOAt):
1. Fmoc-Lys(Mtt)-OH : 1.12 g in 6 ml
2. Fmoc-OEG-OH : 1.39 g in 12 ml (2 couplings)
3. Fmoc-Glu-OtBu : 0.77 g in 6 ml
4. Fmoc-Thx-OH : 0.68 g in 6 ml
5. C-16 diacid mono t-butylester (see WO 2005/012347 A2 example 4): 0.62 g in 6 ml All couplings were performed by adding DIC 7 eq.

After these couplings, the resin was removed from the Liberty synthesizer and was washed, drained, and treated with 5 ml hexafluoro isopropanol for 10 minutes. The resin was then washed with DCM and drained. Then, the hexafluoro isopropanol treatment and DCM washing was repeated.

Suberic acid bis-NHS ester (368 mg) was dissolved in NMP (10 ml, with 0.5 mM bromophenol blue) and DIPEA (170 µl) was added. This solution was added to the drained resin and allowed to react overnight. After the coupling, the resin was washed with NMP, DCM and drained. To the drained resin, a mixture of TFA:TIPS:mercaptoethanol:H₂O 94:1:2.5:2.5 was added and the resin was shaken at ambient temperature for 2 hours. The resin was drained slowly into 75 ml ice-cooled diethyl ether resulting in precipitation of the product. Further stirring at rt. for 0.5 hour and centrifugation yielded the product as a solid which was washed twice with diethylether and dried *in vacuo*. The crude product was dissolved in a mixture of acetonitrile containing HPLC A- and B-buffers, and purified by preparative RP-HPLC using a gradient of acetonitrile/water buffers with 0.1% TFA. Product identity and purity was confirmed by HPLC and LCMS.
Using the above protocol, a highly complex albumin binding side chain was prepared as an NHS ester. This compound is set up to react with GSC in example 17.

### Example 17. Coupling of the NHS ester to GSC to yield substrate 1

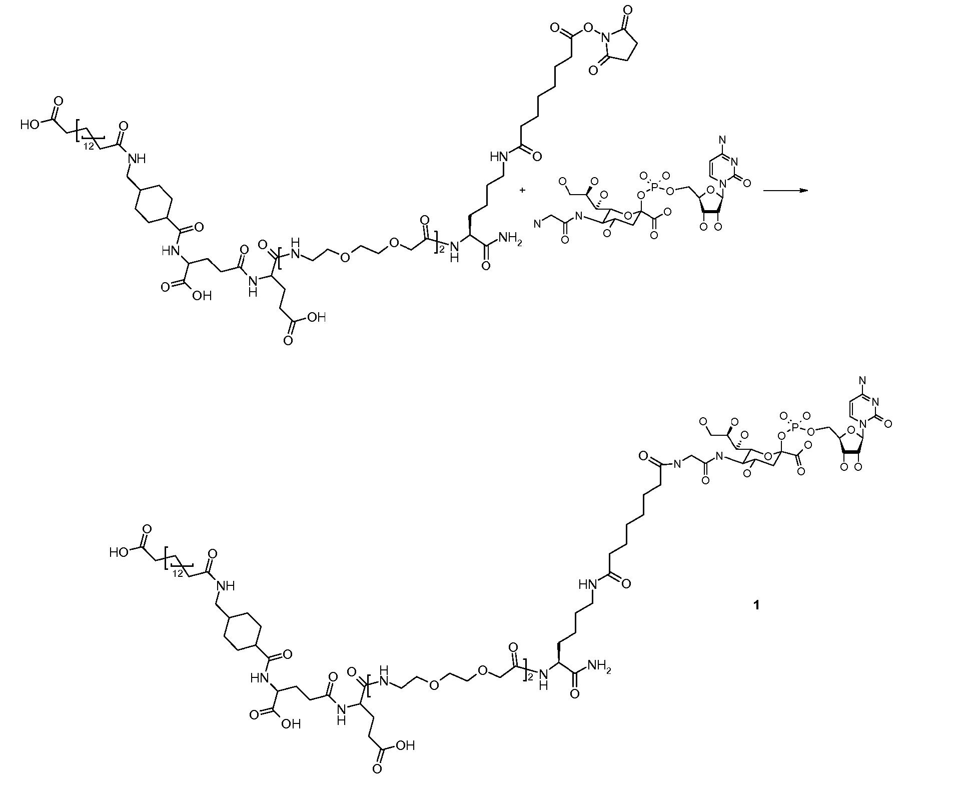

The NHS ester of Example 16 (20 mg) was dissolved in THF (500 µl) and TRIS buffer (100 mM, pH 8.4, 1500 µl) was added. GSC (20 mg) was weighed out and added to the solution of the NHS-ester and allowed to react at rt. for a period of 30 min. The reaction mixture was diluted to 4 ml with water and purified by RP HPLC using a gradient of acetonitrile/water buffers with 0.1% TFA. Gradient 10->50% B-buffer. Relevant fractions were identified by LCMS and freezedried. The product showed excellent water-solubility. Yield: 7.7 mg. The product was identified by LCMS.

Using the above protocol, a sialyl transferase substrate carrying a complex albumin binding hydrophobic side chain was prepared. The substrate 1 proved to be fully soluble in aqueous buffers.

## Claims

1. A method for purification of a protein that is conjugated to an albumin binding moiety from a mixture comprising (i) said protein in said conjugated form and (ii) said protein in a form that is not conjugated to said albumin-binding moiety, the method comprising:
(a) providing a solid support comprising a substance capable of specifically binding to the albumin binding moiety, wherein said substance is selected from the group consisting of albumin and cyclodextrin;
(b) contacting said solid support of (a) with said mixture comprising protein and conjugated protein under suitable conditions for binding of the albumin binding moiety to the substance as defined in (a); and
(c) eluting components bound to the solid support, wherein said elution comprises contacting the solid support with the substance capable of binding to the albumin binding moiety.

2. A method according to claim 1wherein said substance is applied to said solid support in a gradient of increasing concentration in step (c).

3. A method according to any one of the preceding claims wherein the protein is selected from Factor VIII, growth hormone, Factor VII, Factor IX, GLP-1, insulin, or a variant form of any thereof.

4. A method according to any one of the preceding claims wherein the albumin binding moiety extends the half life of the protein *in vivo.*

5. A method according to any one of the preceding claims wherein the albumin binding moiety comprises a fatty acid or fatty acid derivative.

6. A method according to claim 5 wherein the fatty acid derivative comprises a fatty diacid with at least 12 methylene units.

7. A method according to claim 5 or 6wherein the fatty acid or fatty acid derivative is linked to a coagulation factor via a sialic acid of an N-linked glycan.

8. A method according to claim 5 or 6wherein the fatty acid or fatty acid derivative is linked to coagulation factor via a sialic acid of an O-linked glycan.

9. A method according to claim 7 or 8 wherein the coagulation protein is factor VII or factor VIIa.

10. A method according to claim 7 or 8 wherein the coagulation protein is factor VIII.

11. A method according to claim 7 or 8 wherein the coagulation protein is factor IX.

## Patentansprüche

1. Verfahren zur Reinigung eines Proteins, das mit einer albuminbindenden Gruppierung konjugiert ist, aus einem Gemisch, das (i) das Protein in der konjugierten Form und (ii) das Protein in einer Form, die nicht mit der albuminbindenden Gruppierung konjugiert ist, umfasst, wobei das Verfahren Folgendes umfasst:
(a) Bereitstellen eines festen Trägers, der eine Substanz umfasst, die spezifisch an die albumin-bindende Gruppierung binden kann, wobei die Substanz aus der Gruppe bestehend aus Albumin und Cyclodextrin ausgewählt wird;
(b) Inberührungbringen des festen Trägers aus (a) mit dem Gemisch, das Protein und konjugiertes Protein umfasst, unter geeigneten Bedingungen zum Binden der albuminbindenden Gruppierung an die Substanz gemäß (a); und
(c) Eluieren von an den festen Träger gebundenen Komponenten, wobei die Elution das Inberührungbringen des festen Trägers mit der Substanz, die an die albuminbindende Gruppierung binden kann, umfasst.

2. Verfahren nach Anspruch 1, bei dem die Substanz in Schritt (c) in einem Gradienten zunehmender Konzentration auf den festen Träger aufgebracht wird.

3. Verfahren nach einem der vorhergehenden Ansprüche, bei dem das Protein aus Faktor VIII, Wachstumshormon, Faktor VII, Faktor IX, GLP-1, Insulin oder einer varianten Form von einem davon ausgewählt wird.

4. Verfahren nach einem der vorhergehenden Ansprüche, bei dem die albuminbindende Gruppierung die Halbwertszeit des Proteins in vivo verlängert.

5. Verfahren nach einem der vorhergehenden Ansprüche, bei dem die albuminbindende Gruppierung eine Fettsäure oder ein Fettsäurederivat umfasst.

6. Verfahren nach Anspruch 5, bei dem das Fettsäurederivat eine Fettdisäure mit mindestens 12 Methylen-Einheiten umfasst.

7. Verfahren nach Anspruch 5 oder 6, bei dem die Fettsäure bzw. das Fettsäurederivat über eine Sialinsäure eines N-verknüpften Glycans mit einem Koagulationsfaktor verknüpft ist.

8. Verfahren nach Anspruch 5 oder 6, bei dem die Fettsäure bzw. das Fettsäurederivat über eine Sialinsäure eines 0-verknüpften Glycans mit einem Koagulationsfaktor verknüpft ist.

9. Verfahren nach Anspruch 7 oder 8, bei dem es sich bei dem Koagulationsprotein um Faktor VII oder Faktor VIIa handelt.

10. Verfahren nach Anspruch 7 oder 8, bei dem es sich bei dem Koagulationsprotein um Faktor VIII handelt.

11. Verfahren nach Anspruch 7 oder 8, bei dem es sich bei dem Koagulationsprotein um Faktor IX handelt.

## Revendications

1. Procédé de purification d'une protéine qui est conjuguée à un fragment de liaison d'albumine à partir d'un mélange comprenant (i) ladite protéine sous ladite forme conjuguée et (ii) ladite protéine sous une forme qui n'est pas conjuguée audit fragment de liaison d'albumine, le procédé comprenant :
(a) la fourniture d'un support solide comprenant une substance capable de se lier spécifiquement au fragment de liaison d'albumine, où ladite substance est choisie dans le groupe constitué de l'albumine et de la cyclodextrine ;
(b) la mise en contact dudit support solide de (a) avec ledit mélange comprenant une protéine et une protéine conjuguée dans des conditions adaptées pour la liaison du fragment de liaison d'albumine à la substance telle que définie dans (a) ; et
(c) l'élution de composants liés au support solide, où ladite élution comprend la mise en contact du support solide avec la substance capable de se lier au fragment de liaison d'albumine.

2. Procédé selon la revendication 1 dans lequel ladite substance est appliquée sur ledit support solide dans un gradient de concentration croissante dans l'étape (c).

3. Procédé selon l'une quelconque des revendications précédentes dans lequel la protéine est choisie parmi le facteur VIII, une hormone de croissance, le facteur VII, le facteur IX, GLP-1, l'insuline ou une forme variante de l'un quelconque de ceux-ci.

4. Procédé selon l'une quelconque des revendications précédentes dans lequel le fragment de liaison d'albumine prolonge la demi-vie de la protéine *in vivo.*

5. Procédé selon l'une quelconque des revendications précédentes dans lequel le fragment de liaison d'albumine comprend un acide gras ou un dérivé d'acide gras.

6. Procédé selon la revendication 5 dans lequel le dérivé d'acide gras comprend un diacide gras avec au moins 12 motifs méthylène.

7. Procédé selon la revendication 5 ou 6 dans lequel l'acide gras ou dérivé d'acide gras est lié à un facteur de coagulation par l'intermédiaire d'un acide sialique d'un N-glycane.

8. Procédé selon la revendication 5 ou 6 dans lequel l'acide gras ou dérivé diacide gras est lié au facteur de coagulation par l'intermédiaire d'un acide sialique d'un O-glycane.

9. Procédé selon la revendication 7 ou 8 dans lequel la protéine de coagulation est le facteur VII ou le facteur VIIa.

10. Procédé selon la revendication 7 ou 8 dans lequel la protéine de coagulation est le facteur VIII.

11. Procédé selon la revendication 7 ou 8 dans lequel la protéine de coagulation est le facteur IX.


### SEQUENCE LISTING

<110> Novo Nordisk A/S
<120> Purification Method
<130> 8060
<160> 1
<170> PatentIn version 3.5
<210> 1
   <211> 2332
   <212> PRT
   <213> homo sapiens
<400> 1
   Ala Thr Arg Arg Tyr Tyr Leu Gly Ala Val Glu Leu Ser Trp Asp Tyr
   1               5                   10                  15
   Met Gln Ser Asp Leu Gly Glu Leu Pro Val Asp Ala Arg Phe Pro Pro
               20                  25                  30
   Arg Val Pro Lys Ser Phe Pro Phe Asn Thr Ser Val Val Tyr Lys Lys
           35                  40                  45
   Thr Leu Phe Val Glu Phe Thr Asp His Leu Phe Asn Ile Ala Lys Pro
       50                  55                  60
   Arg Pro Pro Trp Met Gly Leu Leu Gly Pro Thr Ile Gln Ala Glu Val
   65                  70                  75                  80
   Tyr Asp Thr Val Val Ile Thr Leu Lys Asn Met Ala Ser His Pro Val
                   85                  90                  95
   Ser Leu His Ala Val Gly Val Ser Tyr Trp Lys Ala Ser Glu Gly Ala
               100                 105                 110
   Glu Tyr Asp Asp Gln Thr Ser Gln Arg Glu Lys Glu Asp Asp Lys Val
           115                 120                 125
   Phe Pro Gly Gly Ser His Thr Tyr Val Trp Gln Val Leu Lys Glu Asn
       130                 135                 140
   Gly Pro Met Ala Ser Asp Pro Leu Cys Leu Thr Tyr Ser Tyr Leu Ser
   145                 150                 155                 160
   His Val Asp Leu Val Lys Asp Leu Asn Ser Gly Leu Ile Gly Ala Leu
                   165                 170                 175
   Leu Val Cys Arg Glu Gly Ser Leu Ala Lys Glu Lys Thr Gln Thr Leu
               180                 185                 190
   His Lys Phe Ile Leu Leu Phe Ala Val Phe Asp Glu Gly Lys Ser Trp
   195 200 205
   His Ser Glu Thr Lys Asn Ser Leu Met Gln Asp Arg Asp Ala Ala Ser
   210 215 220
   Ala Arg Ala Trp Pro Lys Met His Thr Val Asn Gly Tyr Val Asn Arg
   225 230 235 240
   Ser Leu Pro Gly Leu Ile Gly Cys His Arg Lys Ser Val Tyr Trp His
   245 250 255
   Val Ile Gly Met Gly Thr Thr Pro Glu Val His Ser Ile Phe Leu Glu
   260 265 270
   Gly His Thr Phe Leu Val Arg Asn His Arg Gln Ala Ser Leu Glu Ile
   275 280 285
   Ser Pro Ile Thr Phe Leu Thr Ala Gln Thr Leu Leu Met Asp Leu Gly
   290 295 300
   Gln Phe Leu Leu Phe Cys His Ile Ser Ser His Gln His Asp Gly Met
   305 310 315 320
   Glu Ala Tyr Val Lys Val Asp Ser Cys Pro Glu Glu Pro Gln Leu Arg
   325 330 335
   Met Lys Asn Asn Glu Glu Ala Glu Asp Tyr Asp Asp Asp Leu Thr Asp
   340 345 350
   Ser Glu Met Asp Val Val Arg Phe Asp Asp Asp Asn Ser Pro Ser Phe
   355 360 365
   Ile Gln Ile Arg Ser Val Ala Lys Lys His Pro Lys Thr Trp Val His
   370 375 380
   Tyr Ile Ala Ala Glu Glu Glu Asp Trp Asp Tyr Ala Pro Leu Val Leu
   385 390 395 400
   Ala Pro Asp Asp Arg Ser Tyr Lys Ser Gln Tyr Leu Asn Asn Gly Pro
   405 410 415
   Gln Arg Ile Gly Arg Lys Tyr Lys Lys Val Arg Phe Met Ala Tyr Thr
   420 425 430
   Asp Glu Thr Phe Lys Thr Arg Glu Ala Ile Gln His Glu Ser Gly Ile
   435 440 445
   Leu Gly Pro Leu Leu Tyr Gly Glu Val Gly Asp Thr Leu Leu Ile Ile
   450 455 460
   Phe Lys Asn Gln Ala Ser Arg Pro Tyr Asn Ile Tyr Pro His Gly Ile
   465 470 475 480
   Thr Asp Val Arg Pro Leu Tyr Ser Arg Arg Leu Pro Lys Gly Val Lys
   485 490 495
   His Leu Lys Asp Phe Pro Ile Leu Pro Gly Glu Ile Phe Lys Tyr Lys
   500 505 510
   Trp Thr Val Thr Val Glu Asp Gly Pro Thr Lys Ser Asp Pro Arg Cys
   515 520 525
   Leu Thr Arg Tyr Tyr Ser Ser Phe Val Asn Met Glu Arg Asp Leu Ala
   530 535 540
   Ser Gly Leu Ile Gly Pro Leu Leu Ile Cys Tyr Lys Glu Ser Val Asp
   545 550 555 560
   Gln Arg Gly Asn Gln Ile Met Ser Asp Lys Arg Asn Val Ile Leu Phe
   565 570 575
   Ser Val Phe Asp Glu Asn Arg Ser Trp Tyr Leu Thr Glu Asn Ile Gln
   580 585 590
   Arg Phe Leu Pro Asn Pro Ala Gly Val Gln Leu Glu Asp Pro Glu Phe
   595 600 605
   Gln Ala Ser Asn Ile Met His Ser Ile Asn Gly Tyr Val Phe Asp Ser
   610 615 620
   Leu Gln Leu Ser Val Cys Leu His Glu Val Ala Tyr Trp Tyr Ile Leu
   625 630 635 640
   Ser Ile Gly Ala Gln Thr Asp Phe Leu Ser Val Phe Phe Ser Gly Tyr
   645 650 655
   Thr Phe Lys His Lys Met Val Tyr Glu Asp Thr Leu Thr Leu Phe Pro
   660 665 670
   Phe Ser Gly Glu Thr Val Phe Met Ser Met Glu Asn Pro Gly Leu Trp
   675 680 685
   Ile Leu Gly Cys His Asn Ser Asp Phe Arg Asn Arg Gly Met Thr Ala
   690 695 700
   Leu Leu Lys Val Ser Ser Cys Asp Lys Asn Thr Gly Asp Tyr Tyr Glu
   705 710 715 720
   Asp Ser Tyr Glu Asp Ile Ser Ala Tyr Leu Leu Ser Lys Asn Asn Ala
   725 730 735
   Ile Glu Pro Arg Ser Phe Ser Gln Asn Ser Arg His Pro Ser Thr Arg
   740 745 750
   Gln Lys Gln Phe Asn Ala Thr Thr Ile Pro Glu Asn Asp Ile Glu Lys
   755 760 765
   Thr Asp Pro Trp Phe Ala His Arg Thr Pro Met Pro Lys Ile Gln Asn
   770 775 780
   Val Ser Ser Ser Asp Leu Leu Met Leu Leu Arg Gln Ser Pro Thr Pro
   785 790 795 800
   His Gly Leu Ser Leu Ser Asp Leu Gln Glu Ala Lys Tyr Glu Thr Phe
   805 810 815
   Ser Asp Asp Pro Ser Pro Gly Ala Ile Asp Ser Asn Asn Ser Leu Ser
   820 825 830
   Glu Met Thr His Phe Arg Pro Gln Leu His His Ser Gly Asp Met Val
   835 840 845
   Phe Thr Pro Glu Ser Gly Leu Gln Leu Arg Leu Asn Glu Lys Leu Gly
   850 855 860
   Thr Thr Ala Ala Thr Glu Leu Lys Lys Leu Asp Phe Lys Val Ser Ser
   865 870 875 880
   Thr Ser Asn Asn Leu Ile Ser Thr Ile Pro Ser Asp Asn Leu Ala Ala
   885 890 895
   Gly Thr Asp Asn Thr Ser Ser Leu Gly Pro Pro Ser Met Pro Val His
   900 905 910
   Tyr Asp Ser Gln Leu Asp Thr Thr Leu Phe Gly Lys Lys Ser Ser Pro
   915 920 925
   Leu Thr Glu Ser Gly Gly Pro Leu Ser Leu Ser Glu Glu Asn Asn Asp
   930 935 940
   Ser Lys Leu Leu Glu Ser Gly Leu Met Asn Ser Gln Glu Ser Ser Trp
   945 950 955 960
   Gly Lys Asn Val Ser Ser Thr Glu Ser Gly Arg Leu Phe Lys Gly Lys
   965 970 975
   Arg Ala His Gly Pro Ala Leu Leu Thr Lys Asp Asn Ala Leu Phe Lys
   980 985 990
   Val Ser Ile Ser Leu Leu Lys Thr Asn Lys Thr Ser Asn Asn Ser Ala
   995 1000 1005
   Thr Asn Arg Lys Thr His Ile Asp Gly Pro Ser Leu Leu Ile Glu
   1010 1015 1020
   Asn Ser Pro Ser Val Trp Gln Asn Ile Leu Glu Ser Asp Thr Glu
   1025 1030 1035
   Phe Lys Lys Val Thr Pro Leu Ile His Asp Arg Met Leu Met Asp
   1040 1045 1050
   Lys Asn Ala Thr Ala Leu Arg Leu Asn His Met Ser Asn Lys Thr
   1055 1060 1065
   Thr Ser Ser Lys Asn Met Glu Met Val Gln Gln Lys Lys Glu Gly
   1070 1075 1080
   Pro Ile Pro Pro Asp Ala Gln Asn Pro Asp Met Ser Phe Phe Lys
   1085 1090 1095
   Met Leu Phe Leu Pro Glu Ser Ala Arg Trp Ile Gln Arg Thr His
   1100 1105 1110
   Gly Lys Asn Ser Leu Asn Ser Gly Gln Gly Pro Ser Pro Lys Gln
   1115 1120 1125
   Leu Val Ser Leu Gly Pro Glu Lys Ser Val Glu Gly Gln Asn Phe
   1130 1135 1140
   Leu Ser Glu Lys Asn Lys Val Val Val Gly Lys Gly Glu Phe Thr
   1145 1150 1155
   Lys Asp Val Gly Leu Lys Glu Met Val Phe Pro Ser Ser Arg Asn
   1160 1165 1170
   Leu Phe Leu Thr Asn Leu Asp Asn Leu His Glu Asn Asn Thr His
   1175 1180 1185
   Asn Gln Glu Lys Lys Ile Gln Glu Glu Ile Glu Lys Lys Glu Thr
   1190 1195 1200
   Leu Ile Gln Glu Asn Val Val Leu Pro Gln Ile His Thr Val Thr
   1205 1210 1215
   Gly Thr Lys Asn Phe Met Lys Asn Leu Phe Leu Leu Ser Thr Arg
   1220 1225 1230
   Gln Asn Val Glu Gly Ser Tyr Asp Gly Ala Tyr Ala Pro Val Leu
   1235 1240 1245
   Gln Asp Phe Arg Ser Leu Asn Asp Ser Thr Asn Arg Thr Lys Lys
   1250 1255 1260
   His Thr Ala His Phe Ser Lys Lys Gly Glu Glu Glu Asn Leu Glu
   1265 1270 1275
   Gly Leu Gly Asn Gln Thr Lys Gln Ile Val Glu Lys Tyr Ala Cys
   1280 1285 1290
   Thr Thr Arg Ile Ser Pro Asn Thr Ser Gln Gln Asn Phe Val Thr
   1295 1300 1305
   Gln Arg Ser Lys Arg Ala Leu Lys Gln Phe Arg Leu Pro Leu Glu
   1310 1315 1320
   Glu Thr Glu Leu Glu Lys Arg Ile Ile Val Asp Asp Thr Ser Thr
   1325 1330 1335
   Gln Trp Ser Lys Asn Met Lys His Leu Thr Pro Ser Thr Leu Thr
   1340 1345 1350
   Gln Ile Asp Tyr Asn Glu Lys Glu Lys Gly Ala Ile Thr Gln Ser
   1355 1360 1365
   Pro Leu Ser Asp Cys Leu Thr Arg Ser His Ser Ile Pro Gln Ala
   1370 1375 1380
   Asn Arg Ser Pro Leu Pro Ile Ala Lys Val Ser Ser Phe Pro Ser
   1385 1390 1395
   Ile Arg Pro Ile Tyr Leu Thr Arg Val Leu Phe Gln Asp Asn Ser
   1400 1405 1410
   Ser His Leu Pro Ala Ala Ser Tyr Arg Lys Lys Asp Ser Gly Val
   1415 1420 1425
   Gln Glu Ser Ser His Phe Leu Gln Gly Ala Lys Lys Asn Asn Leu
   1430 1435 1440
   Ser Leu Ala Ile Leu Thr Leu Glu Met Thr Gly Asp Gln Arg Glu
   1445 1450 1455
   Val Gly Ser Leu Gly Thr Ser Ala Thr Asn Ser Val Thr Tyr Lys
   1460 1465 1470
   Lys Val Glu Asn Thr Val Leu Pro Lys Pro Asp Leu Pro Lys Thr
   1475 1480 1485
   Ser Gly Lys Val Glu Leu Leu Pro Lys Val His Ile Tyr Gln Lys
   1490 1495 1500
   Asp Leu Phe Pro Thr Glu Thr Ser Asn Gly Ser Pro Gly His Leu
   1505 1510 1515
   Asp Leu Val Glu Gly Ser Leu Leu Gln Gly Thr Glu Gly Ala Ile
   1520 1525 1530
   Lys Trp Asn Glu Ala Asn Arg Pro Gly Lys Val Pro Phe Leu Arg
   1535 1540 1545
   Val Ala Thr Glu Ser Ser Ala Lys Thr Pro Ser Lys Leu Leu Asp
   1550 1555 1560
   Pro Leu Ala Trp Asp Asn His Tyr Gly Thr Gln Ile Pro Lys Glu
   1565 1570 1575
   Glu Trp Lys Ser Gln Glu Lys Ser Pro Glu Lys Thr Ala Phe Lys
   1580 1585 1590
   Lys Lys Asp Thr Ile Leu Ser Leu Asn Ala Cys Glu Ser Asn His
   1595 1600 1605
   Ala Ile Ala Ala Ile Asn Glu Gly Gln Asn Lys Pro Glu Ile Glu
   1610 1615 1620
   Val Thr Trp Ala Lys Gln Gly Arg Thr Glu Arg Leu Cys Ser Gln
   1625 1630 1635
   Asn Pro Pro Val Leu Lys Arg His Gln Arg Glu Ile Thr Arg Thr
   1640 1645 1650
   Thr Leu Gln Ser Asp Gln Glu Glu Ile Asp Tyr Asp Asp Thr Ile
   1655 1660 1665
   Ser Val Glu Met Lys Lys Glu Asp Phe Asp Ile Tyr Asp Glu Asp
   1670 1675 1680
   Glu Asn Gln Ser Pro Arg Ser Phe Gln Lys Lys Thr Arg His Tyr
   1685 1690 1695
   Phe Ile Ala Ala Val Glu Arg Leu Trp Asp Tyr Gly Met Ser Ser
   1700 1705 1710
   Ser Pro His Val Leu Arg Asn Arg Ala Gln Ser Gly Ser Val Pro
   1715 1720 1725
   Gln Phe Lys Lys Val Val Phe Gln Glu Phe Thr Asp Gly Ser Phe
   1730 1735 1740
   Thr Gln Pro Leu Tyr Arg Gly Glu Leu Asn Glu His Leu Gly Leu
   1745 1750 1755
   Leu Gly Pro Tyr Ile Arg Ala Glu Val Glu Asp Asn Ile Met Val
   1760 1765 1770
   Thr Phe Arg Asn Gln Ala Ser Arg Pro Tyr Ser Phe Tyr Ser Ser
   1775 1780 1785
   Leu Ile Ser Tyr Glu Glu Asp Gln Arg Gln Gly Ala Glu Pro Arg
   1790 1795 1800
   Lys Asn Phe Val Lys Pro Asn Glu Thr Lys Thr Tyr Phe Trp Lys
   1805 1810 1815
   Val Gln His His Met Ala Pro Thr Lys Asp Glu Phe Asp Cys Lys
   1820 1825 1830
   Ala Trp Ala Tyr Phe Ser Asp Val Asp Leu Glu Lys Asp Val His
   1835 1840 1845
   Ser Gly Leu Ile Gly Pro Leu Leu Val Cys His Thr Asn Thr Leu
   1850 1855 1860
   Asn Pro Ala His Gly Arg Gln Val Thr Val Gln Glu Phe Ala Leu
   1865 1870 1875
   Phe Phe Thr Ile Phe Asp Glu Thr Lys Ser Trp Tyr Phe Thr Glu
   1880 1885 1890
   Asn Met Glu Arg Asn Cys Arg Ala Pro Cys Asn Ile Gln Met Glu
   1895 1900 1905
   Asp Pro Thr Phe Lys Glu Asn Tyr Arg Phe His Ala Ile Asn Gly
   1910 1915 1920
   Tyr Ile Met Asp Thr Leu Pro Gly Leu Val Met Ala Gln Asp Gln
   1925 1930 1935
   Arg Ile Arg Trp Tyr Leu Leu Ser Met Gly Ser Asn Glu Asn Ile
   1940 1945 1950
   His Ser Ile His Phe Ser Gly His Val Phe Thr Val Arg Lys Lys
   1955 1960 1965
   Glu Glu Tyr Lys Met Ala Leu Tyr Asn Leu Tyr Pro Gly Val Phe
   1970 1975 1980
   Glu Thr Val Glu Met Leu Pro Ser Lys Ala Gly Ile Trp Arg Val
   1985 1990 1995
   Glu Cys Leu Ile Gly Glu His Leu His Ala Gly Met Ser Thr Leu
   2000 2005 2010
   Phe Leu Val Tyr Ser Asn Lys Cys Gln Thr Pro Leu Gly Met Ala
   2015 2020 2025
   Ser Gly His Ile Arg Asp Phe Gln Ile Thr Ala Ser Gly Gln Tyr
   2030 2035 2040
   Gly Gln Trp Ala Pro Lys Leu Ala Arg Leu His Tyr Ser Gly Ser
   2045 2050 2055
   Ile Asn Ala Trp Ser Thr Lys Glu Pro Phe Ser Trp Ile Lys Val
   2060 2065 2070
   Asp Leu Leu Ala Pro Met Ile Ile His Gly Ile Lys Thr Gln Gly
   2075 2080 2085
   Ala Arg Gln Lys Phe Ser Ser Leu Tyr Ile Ser Gln Phe Ile Ile
   2090 2095 2100
   Met Tyr Ser Leu Asp Gly Lys Lys Trp Gln Thr Tyr Arg Gly Asn
   2105 2110 2115
   Ser Thr Gly Thr Leu Met Val Phe Phe Gly Asn Val Asp Ser Ser
   2120 2125 2130
   Gly Ile Lys His Asn Ile Phe Asn Pro Pro Ile Ile Ala Arg Tyr
   2135 2140 2145
   Ile Arg Leu His Pro Thr His Tyr Ser Ile Arg Ser Thr Leu Arg
   2150 2155 2160
   Met Glu Leu Met Gly Cys Asp Leu Asn Ser Cys Ser Met Pro Leu
   2165 2170 2175
   Gly Met Glu Ser Lys Ala Ile Ser Asp Ala Gln Ile Thr Ala Ser
   2180 2185 2190
   Ser Tyr Phe Thr Asn Met Phe Ala Thr Trp Ser Pro Ser Lys Ala
   2195 2200 2205
   Arg Leu His Leu Gln Gly Arg Ser Asn Ala Trp Arg Pro Gln Val
   2210 2215 2220
   Asn Asn Pro Lys Glu Trp Leu Gln Val Asp Phe Gln Lys Thr Met
   2225 2230 2235
   Lys Val Thr Gly Val Thr Thr Gln Gly Val Lys Ser Leu Leu Thr
   2240 2245 2250
   Ser Met Tyr Val Lys Glu Phe Leu Ile Ser Ser Ser Gln Asp Gly
   2255 2260 2265
   His Gln Trp Thr Leu Phe Phe Gln Asn Gly Lys Val Lys Val Phe
   2270 2275 2280
   Gln Gly Asn Gln Asp Ser Phe Thr Pro Val Val Asn Ser Leu Asp
           2285                    2290                    2295
   Pro Pro  Leu Leu Thr Arg Tyr  Leu Arg Ile His Pro  Gln Ser Trp
       2300                    2305                    2310
   Val His  Gln Ile Ala Leu Arg  Met Glu Val Leu Gly  Cys Glu Ala
       2315                    2320                    2325
   Gln Asp  Leu Tyr
       2330